# EUROPEAN PATENT SPECIFICATION

(11) **EP 2 437 879 B1**
(45) Date of publication and mention of the grant of the patent: **26.07.2017**
(21) Application number: 10783680.1
(22) Date of filing: 07.06.2010
(51) Int. Cl.: A01N 25/28, A61K 9/50, B01J 13/12, C09B 67/00

(54) **MULTI-PHASE MICROPARTICLES AND METHOD OF MANUFACTURING MULTI-PHASE MICROPARTICLES**
MEHRPHASENMIKROPARTIKEL UND VERFAHREN ZUR HERSTELLUNG VON MEHRPHASENMIKROPARTIKELN
MICROPARTICULES MULTIPHASE ET LEUR PROCEDE DE FABRICATION

(30) Priority: 05.06.2009 US 184559 P
(43) Date of publication of application: 11.04.2012
(73) Proprietor: Nanyang Technological University, Singapore 639798 (SG)
(72) Inventor: LOO, Say Chye Joachim, Singapore 639798 (SG); LEE, Wei Li, Singapore 639798 (SG)
(74) Representative: Viering, Jentschura & Partner mbB Patent- und Rechtsanwälte
(86) International application number: PCT/SG2010/000215
(87) International publication number: WO 2010/140987

(56) References cited:
- EP-A1- 1 421 990
- WO-A1-2009/075652
- US-A- 5 985 354
- US-A- 6 120 807
- US-A1- 2006 051 425
- SHI M ET AL: "Double walled POE/PLGA microspheres: encapsulation of water-soluble and water-insoluble proteins and their release properties", JOURNAL OF CONTROLLED RELEASE, ELSEVIER, AMSTERDAM, NL, vol. 89, no. 2, 29 April 2003 (2003-04-29) , pages 167-177, XP004421353, ISSN: 0168-3659, DOI: 10.1016/S0168-3659(02)00493-5
- FREITAS S ET AL: "Microencapsulation by solvent extraction/evaporation: reviewing the state of the art of microsphere preparation process technology", JOURNAL OF CONTROLLED RELEASE, ELSEVIER, AMSTERDAM, NL, vol. 102, no. 2, 2 February 2005 (2005-02-02), pages 313-332, XP027664488, ISSN: 0168-3659 [retrieved on 2005-02-02]
- HIGUCHI T. ET AL: 'Spontaneous formation of polymer nanoparticle with inner microphase separation structure' SOFT MATTER vol. 4, 2008, pages 1302 - 1305, XP055036120
- ANA SARAIVA ET AL: "An experimental investigation of cloud-point curves for the poly(ethylene glycol)/water system at varying molecular weight distributions", FLUID PHASE EQUILIBRIA, vol. 91, no. 2, 15 December 1993 (1993-12-15), pages 291-311, XP055260285, AMSTERDAM, NL ISSN: 0378-3812, DOI: 10.1016/0378-3812(93)85105-U

## Description

### FIELD OF THE INVENTION

The present invention refers to the field of polymer chemistry in particular the field of polymer chemistry for the manufacture of particulate material for sustained release of substances.

### BACKGROUND OF THE INVENTION

Controlled delivery of substances such as drugs, insecticides, fertilizers indicators and other active compounds can be achieved by a variety of processes. In one type of delivery system, a polymeric particle is formed, in which a single polymer layer incorporates the active compound to be delivered. When used for drug delivery, this type of delivery system can achieve controlled drug release by protecting drugs from degradation before the release site is reached in the human body.

However, such single-layered (or single-walled) microparticles have limitations, such as inability to provide constant (zero order) drug release and a lack of time-delayed or pulsatile release of therapeutic agents. Therefore, double-layered (or double-walled) microparticles have been developed to provide greater versatility in controlling drug release kinetics and profile.

EP 1 421 990 A1 discloses microcapsules for encapsulating enzymes made of polyols, such as PEG 400, PEG 8000 and PCL, obtained by dispersing the polyols and the enzyme into an organic solvent, emulsifying the thus obtained solution in an aqueous solvent and solidifying the microcapsules.

It is an object of the present invention to provide alternative delivery systems which can be used for the delivery and/or sustained release of substances.

### SUMMARY OF THE INVENTION

In a first aspect, the present invention refers to a method of manufacturing multi-phase microparticles according to claim 1. The method comprises dissolving at least three different polymers in a volatile organic solvent to obtain a first solution. The first solution comprises at least two cloud points, wherein the second cloud point (CP2) is higher than the first cloud point (CP1). Viscosity of the first solution, the first cloud point (CP1) and the second cloud point (CP2) are selected such that the at least three different polymers become immiscible with each other upon evaporation of the volatile organic solvent. The first solution is dispersed into an aqueous continuous phase which comprises a surfactant to obtain an emulsion. The volatile organic solvent is evaporated from the emulsion. The total concentration of the at least three different polymers together in the emulsion before evaporation is below the first cloud point, or is above the first cloud point and below the second cloud point or is above the second cloud point.

In a second aspect, the present invention refers to a multi-phase microparticle obtained by a method of the present invention.

In a third aspect, the present invention refers to a pharmaceutical composition comprising a multi-phase microparticle obtained by a method of the present invention.

### BRIEF DESCRIPTION OF THE DRAWINGS

The invention will be better understood with reference to the detailed description when considered in conjunction with the non-limiting examples and the accompanying drawings, in which:

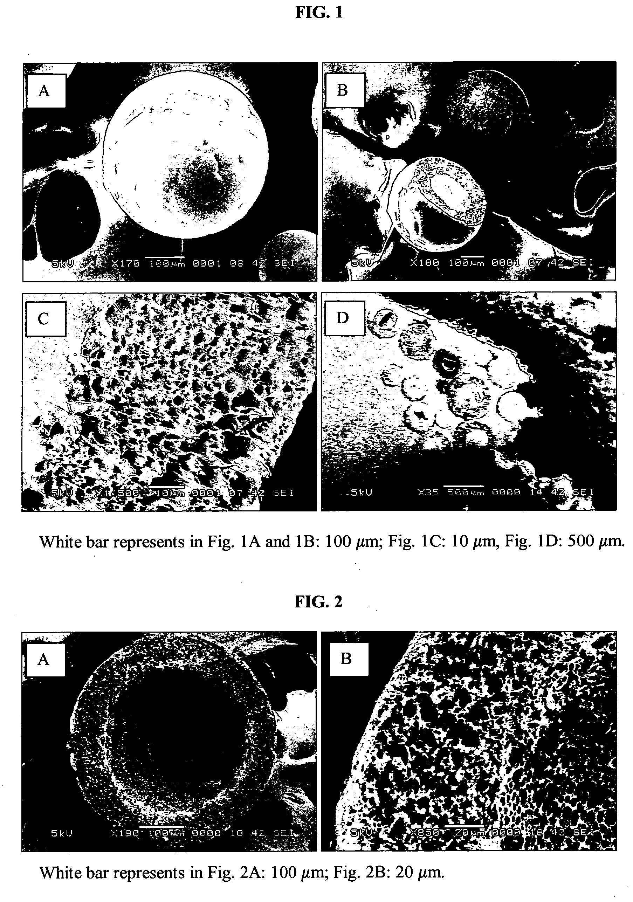

**Figure 1** shows scanning electron microscopy (SEM) micrographs of ternary phase poly(L-lactic acid)/poly(DL-lactic-co-glycolic acid/poly(caprolactone) (PLLA/PLGA/PCL) composite dual-walled (DW) microparticle. **Figure 1A** is a micrograph of an external morphology of the microparticle. **Figure 1B** is a micrograph of a cross-sectional view of the microparticle. **Figure 1C** is a micrograph of a close-up view of the internal morphology of the microparticle. **Figure 1D** is a micrograph of a cross-sectional view of the microparticles embedded in a polyacrylic resin.
**Figure 2** shows SEM micrographs of ternary phase PLLA/PLGA/PCL composite dual-walled microparticles after dissolution with tetrahydrofuran (THF). **Figure 2A** is a micrograph showing cross-sectional view of a microparticle. **Figure 2B** is a micrograph showing a close-up view of the porous shell

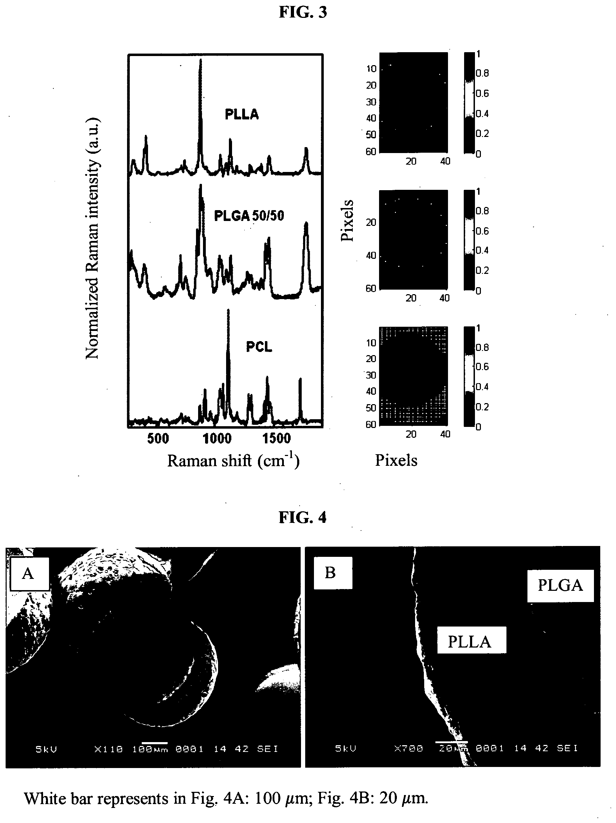

**Figure 3** shows pure component Raman spectra estimates and their associated score images obtained via BTEM from a ternary phase PLLA/PLGA/PCL dual-walled composite microparticle. The axes of score images are in pixels and can be directly correlated to distance by multiplying each pixel with 5 µm.
**Figure 4** shows SEM micrographs of ternary phase PLLA/PLGA dual-walled microsphere. **Figure 4A** is a micrograph showing cross-sectional view of a microparticle. **Figure 4B** is a micrograph showing a close-up view of the shell.

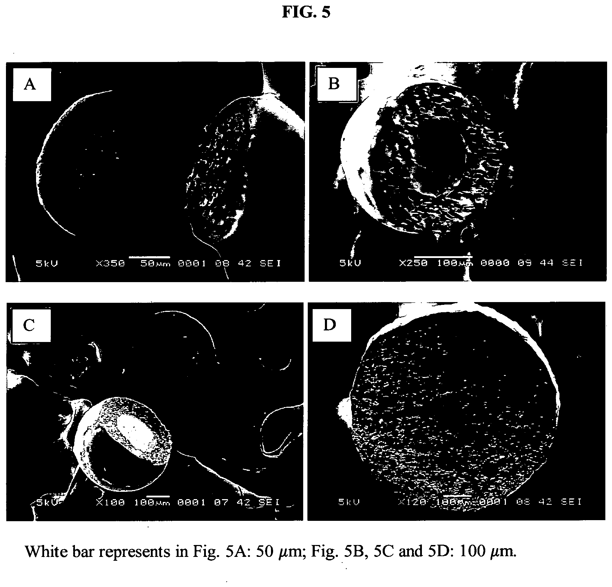

**Figure 5** shows SEM micrographs of ternary phase PLLA/PLGA/PCL microparticles prepared using various polymer solution concentration of (A) 2% (w/v), (B) 4%(w/v), (C) 6% (w/v), and (D) 10% (w/v).

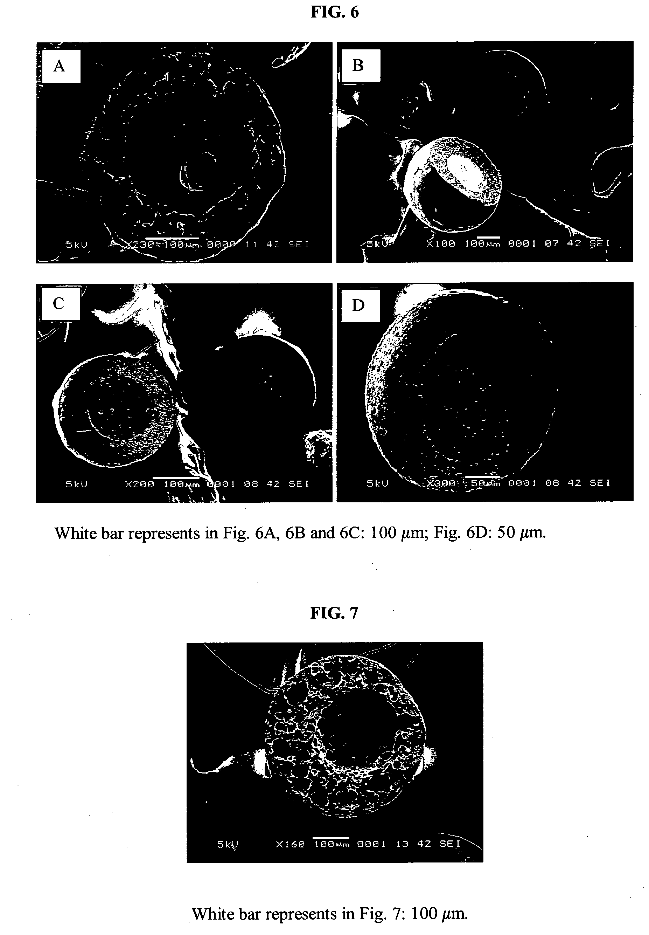

**Figure 6** shows SEM micrographs of ternary phase PLLA/PLGA/PCL microparticles prepared using various stirring speed of (A) 150 rpm, (B) 300 rpm, (C) 400 rpm, and (D) 500 rpm.
**Figure 7** is a SEM micrograph of ternary phase PLLA/PLGA/PCL microparticle prepared at a stirring speed of 150 rpm after dissolution with THF.

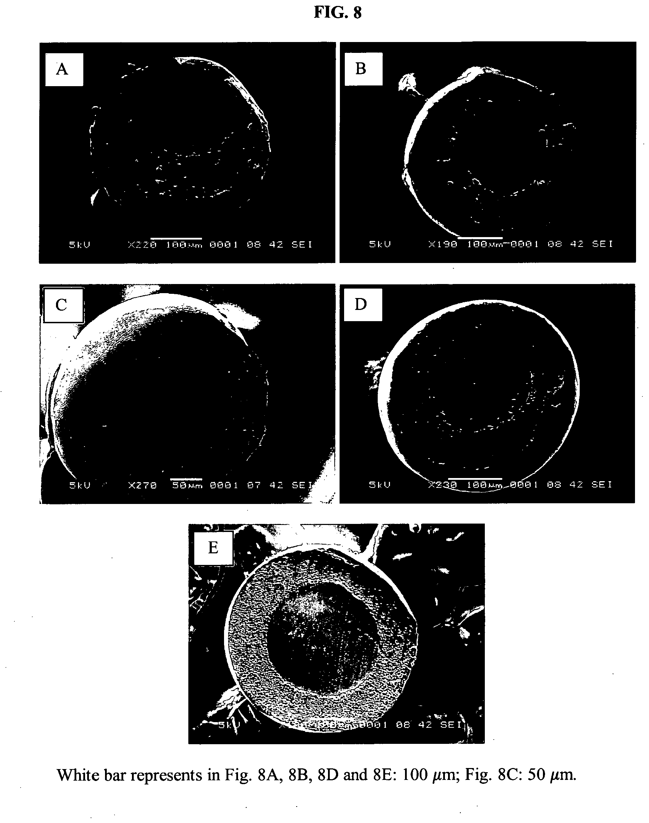

**Figure 8** shows SEM micrographs of ternary phase PLLA/PLGA/PCL microparticles prepared using various volume of poly(vinyl alcohol) (PVA) aqueous solution of (A) 50 mL (oil/water (o/w): 0.1), (B) 150 mL (o/w: 0.03), (C) 250 mL (o/w: 0.02), (D) 400 mL (o/w: 0.0125), and (E) 600 mL (o/w: 0.0083).

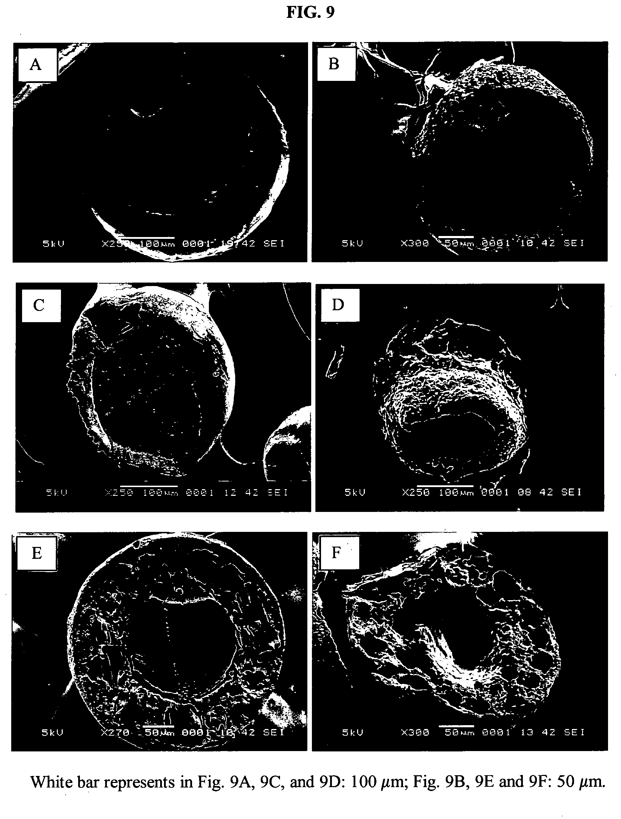

**Figure 9** shows SEM micrographs of microspheres prepared using various mass ratio of ternary phase PLLA:PLGA:PCL of (A) 2 : 3 : 1 , (C) 1 : 3 : 1, and (E) 3 : 2 : 2. Figure 9B, 9D and 9F are the corresponding cross-sectional views after dissolution with THF.

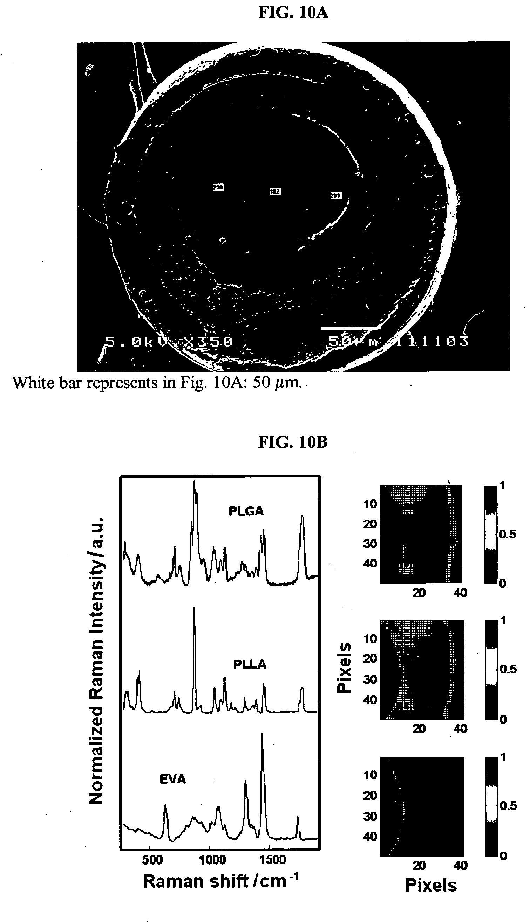

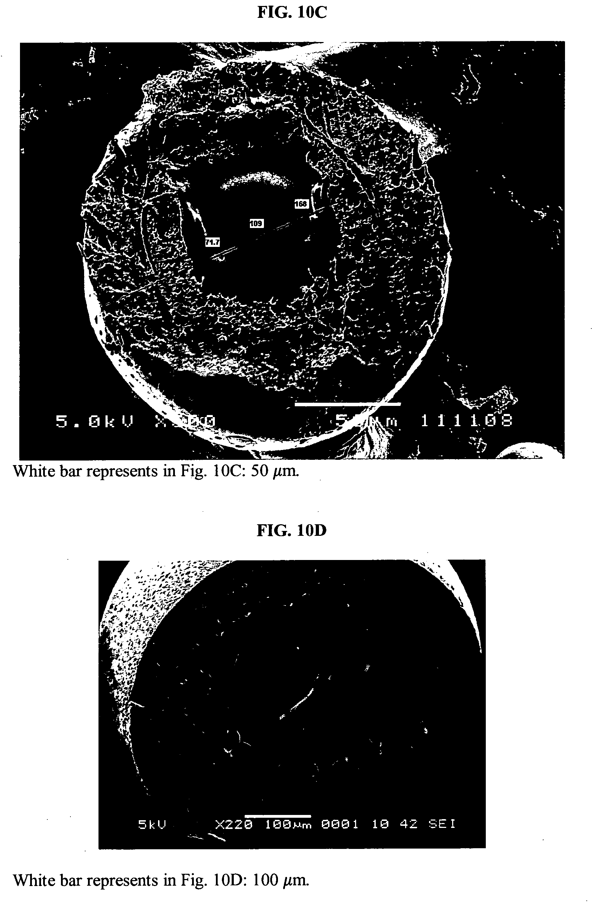

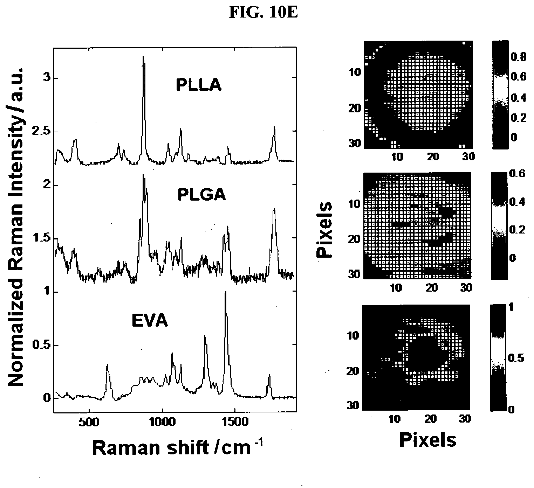

**Figure 10A** is a SEM micrograph of a triple-walled (TW) microsphere of ternary phase poly(DL-lactic-co-glycolic acid/poly(L-lactic acid)/ethylene vinyl acetate (PLGA/PLLA/EVA), corresponding to the outermost shell, middle layer and core respectively. **Figure 10B** shows pure component Raman spectra estimates and their associated score images obtained via BTEM from the ternary phase PLGA/PLLA/EVA triple-walled composite microparticle. **Figure 10C** is a SEM micrograph of a triple-walled microsphere of PLGA/PLLA/EVA, in which the PLGA shell has a larger thickness. **Figure 10D** shows a SEM micrograph of a layer-inverted ternary phase PLLA/PLGA/EVA microsphere, in which the outermost shell, middle layer, and core are PLLA, PLGA, and EVA respectively. **Figure 10E** shows pure component Raman spectra estimates and their associated score images obtained via BTEM from the ternary phase PLLA/PLGA/EVA triple-walled composite microparticle.

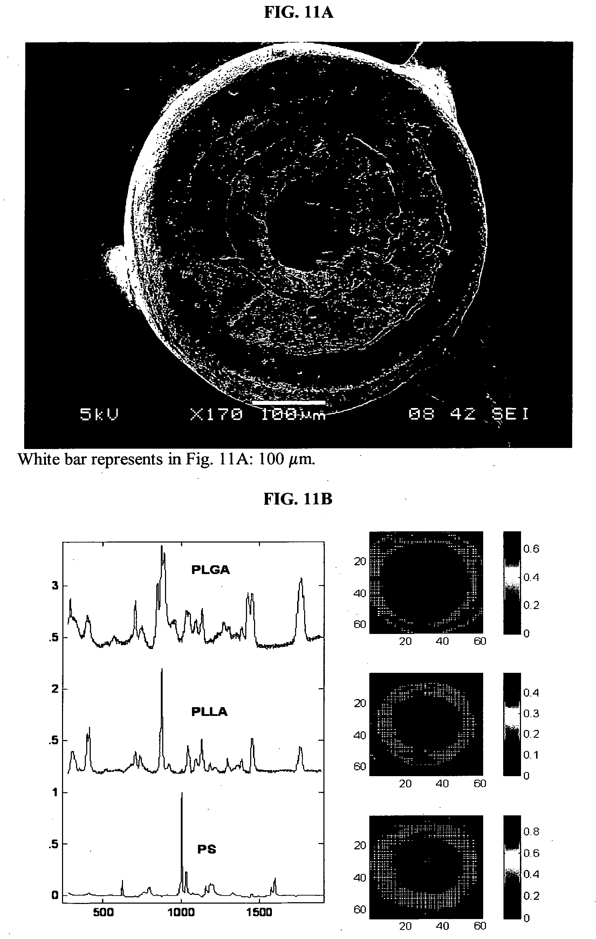

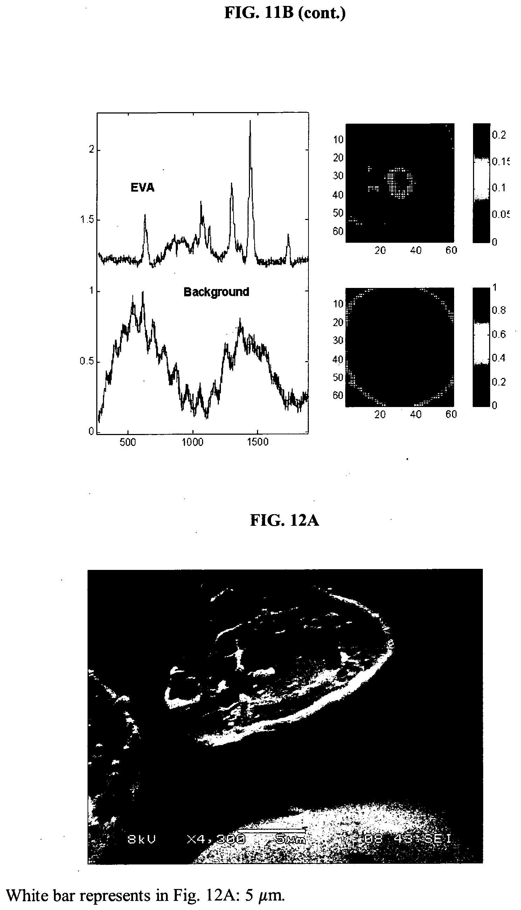

**Figure 11A** is a SEM micrograph of a four-layered poly(DL-lactic-co-glycolic acid/poly(L-lactic acid)/polystyrene/ethylene vinyl acetate (PLGA/PLLA/PS/EVA) microparticle. Shell to core configuration: PLGA, PLLA, PS, EVA. **Figure 11B** shows pure component Raman spectra estimates and their associated score images obtained via BTEM from the PLGA/PLLA/PS/EVA four-layered composite microparticle.

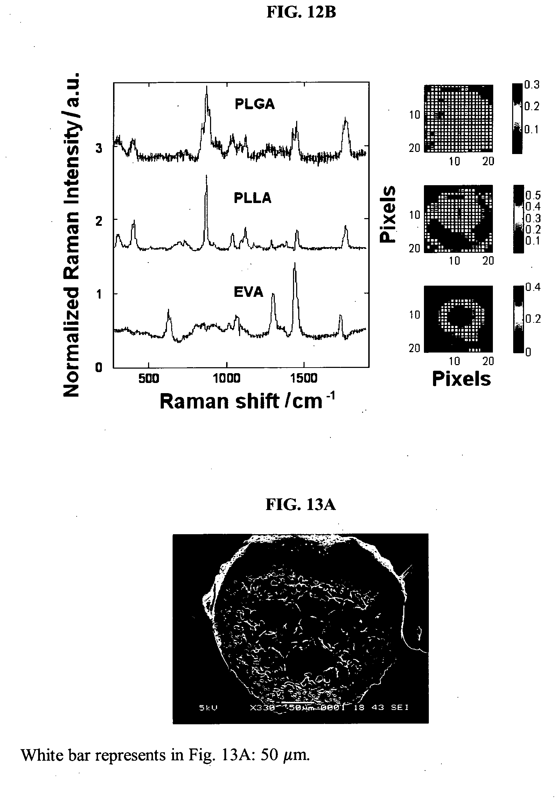

**Figure 12A** is a SEM micrograph of a triple-walled PLGA/PLLA/EVA microparticle, corresponding to the outermost shell, middle layer and core respectively, having a size of about 15 µm. **Figure 12B** shows pure component Raman spectra estimates and their associated score images obtained via BTEM from the PLGA/PLLA/EVA triple-walled composite microparticle.

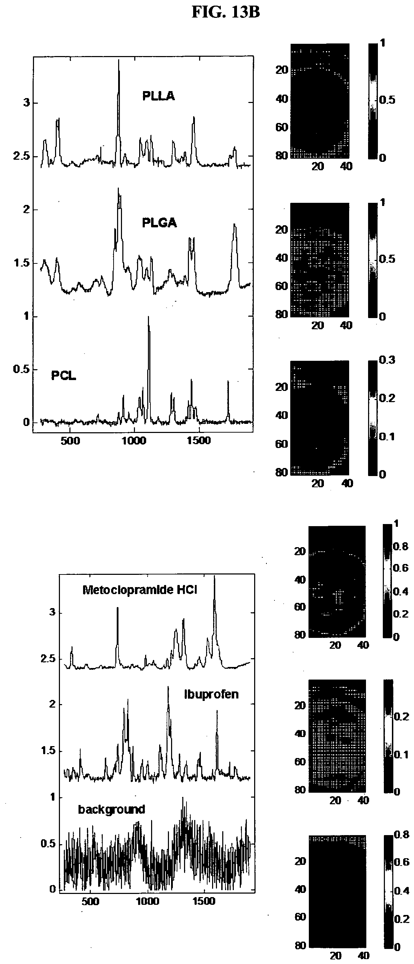

**Figure 13A** is a SEM micrograph of a ternary phase PLLA/PLGA/PCL dual-walled composite microparticle. Ibuprofen (hydrophobic) is localized in the PLLA shell, impregnated with PCL particulates; Metoclopramide HCl (hydrophilic) is localized in the PLGA core. **Figure 13B** shows pure component Raman spectra estimates and their associated score images obtained via BTEM from the microparticle.

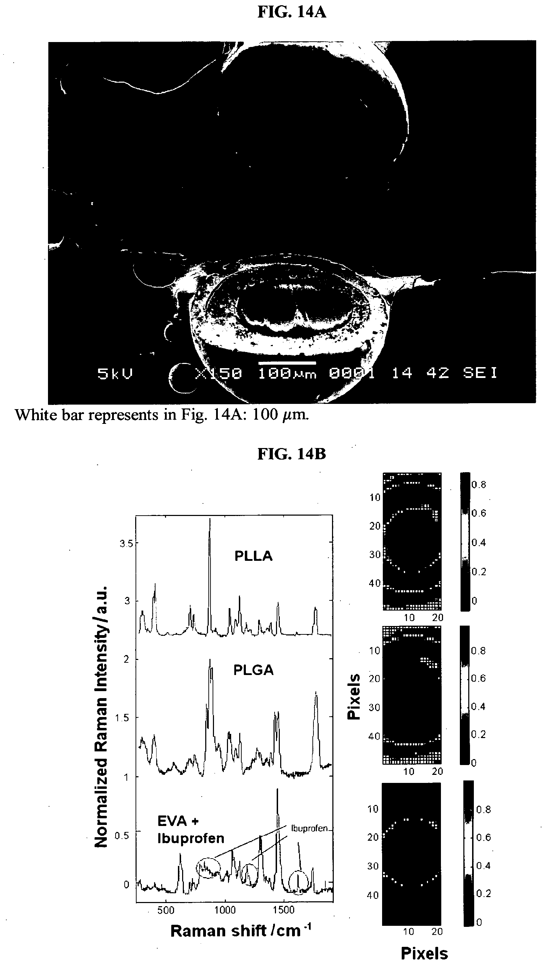

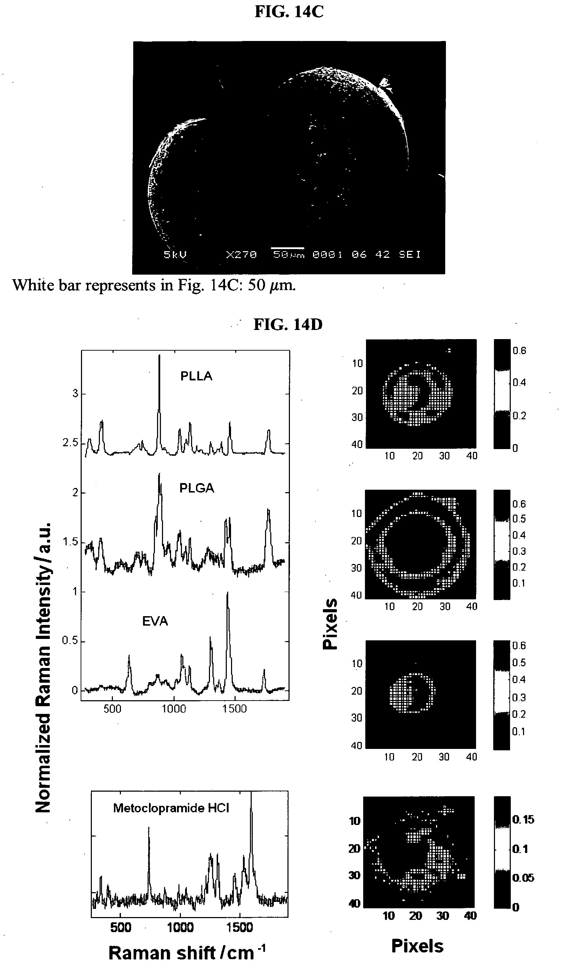

**Figure 14A** is a SEM micrograph of a ternary phase PLGA/PLLA/EVA triple-walled composite microparticle. The outermost shell, middle layer, and core are PLGA, PLLA, and EVA abundant regions respectively. Ibuprofen (hydrophobic) is localized in the EVA core. **Figure 14B** shows pure component Raman spectra estimates and their associated score images obtained via BTEM from the microparticle. **Figure 14C** is a SEM micrograph of a ternary phase PLGA/PLLA/EVA triple-walled composite microparticle. The outermost shell, middle layer, and core are PLGA, PLLA, and EVA abundant regions, respectively. Metoclopramide HCl (hydrophilic) is localized in the PLGA shell. **Figure 14D** shows pure component Raman spectra estimates and their associated score images obtained via BTEM from the microparticle.

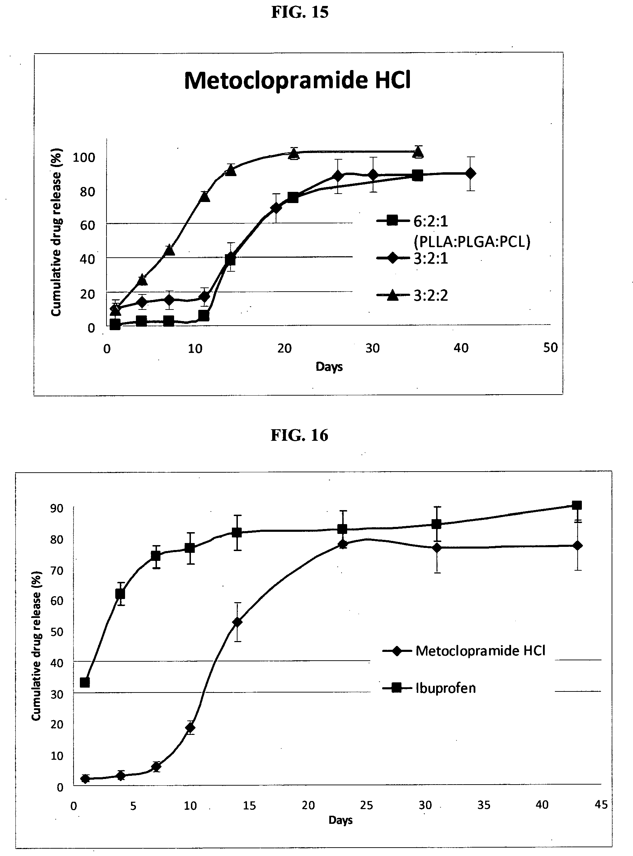

**Figure 15** is a graph showing drug release profiles of ternary phase dual-walled PLLA/PLGA/PCL microparticles with mass ratios of A) 6:2:1, B) 3:2:1, and C) 3:2:2. The shell and core are PLLA impregnated with PCL particulates, and PLGA abundant regions respectively. Metoclopramide HCl (hydrophilic) is localized in the PLGA core. From the graph, it can be seen that the drug release profile of the microparticles can be controlled through changing the polymer mass ratio.
**Figure 16** is a graph showing drug release profiles of a ternary phase dual-walled PLLA/PLGA/PCL microparticle. Metoclopramide HCl and Ibuprofen localized in the PLGA core and PLLA/PCL shell, respectively. From the graph, it can be seen that the release of Ibuprofen was achieved in the first 10 days, followed by Metoclopramide HCl, providing multiple drug release in a sequential manner from a single particle.

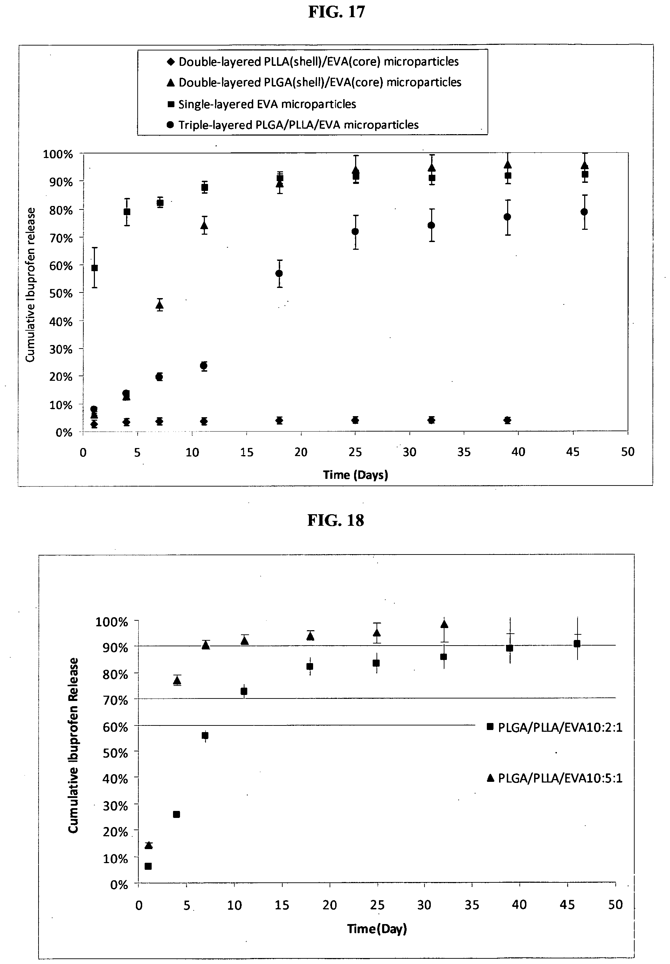

**Figure 17** is a graph showing drug release profiles of (A) double-layered PLLA (shell)/EVA (core) microparticles, (B) double-layered PLGA (shell)/EVA (core) microparticles, (C) single-layered EVA microparticles, and (D) triple-layered PLGA(outer shell) /PLLA (middle layer) /EVA (core) microparticles. From the graph, it can be seen that the triple-layered microparticles achieved a more consistent (close to zero-order) release rate.
**Figure 18** is a graph showing drug release profiles of triple layered PLGA/PLLA/EVA microparticles having mass ratio of (A) 10:2:1, and (B) 10:5:1. Ibuprofen (15 wt%) is localized in the EVA core. The graph shows that drug release profiles can be controlled through changing the polymer mass ratio (i.e. layer thickness).

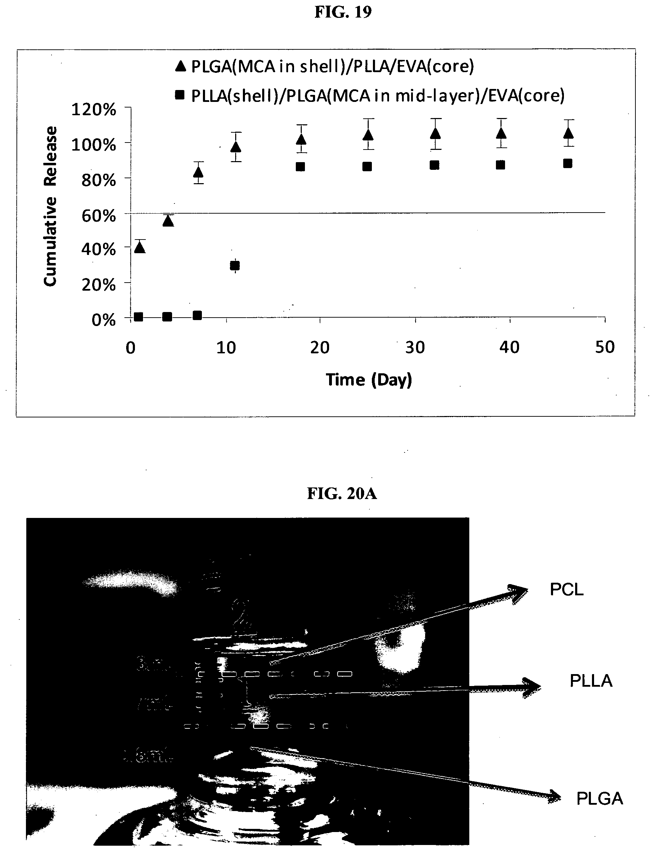

**Figure 19** is a graph showing drug release profiles of triple-layered of microparticles (A) PLGA (MCA in shell)/PLLA/EVA(core) and (B) PLLA (shell)/PLGA(MCA in mid-layer)/EVA(core). The graph shows that drug release profiles can be controlled through layer inversion.

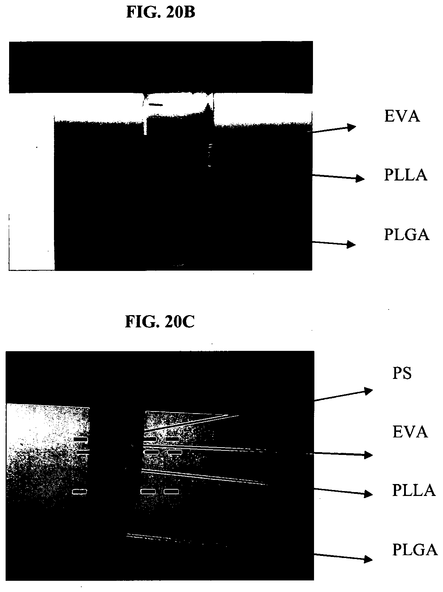

**Figure 20A** is a photograph showing a PLGA/PLLA /PCL solution at a total polymer concentration in solution of 16 % w/v. The first cloud point of the polymer system is 3.75 % w/v and second cloud point of the polymer system is 7 % w/v. Figure 20A shows that the three liquid phases of PLGA, PLLA and PCL separates out and form three distinct phases upon standing, at a total polymer concentration in solution of 16 % w/v, which is greater than the second cloud point of the polymer system at 7 % w/v. First phase separation occurs at 3.75 wt% w/v at which PLGA is phase-separated from PLLA/PCL in solution. Second phase separation occurs at 7 % w/v at which PLLA and PCL phase separates. **Figure 20B** is a photograph showing a PLGA/PLLA/EVA solution with a concentration of 7.5 % w/v. The first cloud point of the polymer system is 4 % w/v and the second cloud point of the polymer system is 5 % w/v. Figure 20B shows that the three liquid phases of PLGA, PLLA and EVA separates out and form three distinct phases upon standing, at a concentration of 7.5 % w/v, which is greater than the second cloud point of the polymer system at 5 % w/v. **Figure 20C** is a photograph showing PLGA/PLLA/PS/EVA solution with a concentration of 13 % w/v. The first cloud point of the polymer system is 4 % w/v, the second cloud point of the polymer system is 5 % w/v and the third cloud point of the polymer system is 12 %w/v. Figure 20C shows that the four liquid phases of PLGA, PLLA and PS and EVA separates out and form four distinct phases at a concentration of 13 % w/v, upon standing, which is greater than the third cloud point of the polymer system at 12 % w/v.

### DETAILED DESCRIPTION OF THE PRESENT INVENTION

The present invention is based on the finding that a multi-phase microparticle can be formed by dissolving at least three different types of polymer in a volatile organic solvent, with subsequent dispersion of the polymer-containing solvent in an aqueous medium, thereby forming an emulsion. The at least three different types of polymer can be selected such that they become immiscible with each other upon evaporation of the volatile organic solvent in which they are dissolved. The solvent can be removed by evaporation from the emulsion. Change in polymer solution concentration as a result of solvent removal causes the polymers to separate in different phases in a sequential manner when they reach the respective cloud point. Accordingly, a multi-phase microparticle comprising discrete regions of different polymer types within the particle can be formed. The configuration of the microparticle, i.e. distribution and size of discrete regions of the different polymer types, can be adapted for specific applications. For example, the multi-phase microparticle can be used for delivery and sustained release of target substances. In particular, the discrete regions of the microparticle can be used to contain different target substances that can be made to release in a controlled manner in application.

In a first aspect, the present invention refers to a method of manufacturing multi-phase microparticles according to the appended claims. The term "multi-phase" as used herein refers to a system having at least three polymer components. Accordingly, the term "multi-phase microparticle" refers to micrometer size particle formed from at least three polymer components, whereby each polymer type occupies discrete regions within the particle. The multi-phase microparticle can also be termed a ternary-phase microparticle when the number of polymer components is three. The ternary-phase microparticle can have two layers or three layers. When the number of polymer components is four, the multi-phase microparticle can be termed a quaternary-phase microparticle. The quaternary-phase microparticle can have two, three or four layers. Each layer in the microparticle can be formed from a different polymer type. A polymer is a large molecule that is built up by repetition of smaller chemical units. The repeat unit of the polymer is usually equivalent or nearly equivalent to the monomer, i.e. starting material from which the polymer is formed. The repeat units of the polymer can be connected to one another via covalent bonds. In some cases, the repetition is linear, such as in the case of a polymer chain having a single backbone with no branches. In some cases, the chains are branched or interconnected to form three-dimensional networks.

The polymers can be formed using processes, such as condensation polymerization and chain-reaction polymerization. Typically, in condensation polymerization, reaction takes places between two molecules to produce one larger molecule, with the possible elimination of a small molecule, such as water. The molecules can comprise a functional group, such as a thiol group, a carboxyl group, an amine group and a halide group, through which polymerization reaction takes place. The reaction continues until almost all of one of the reagents is used up. Generally, chain-reaction polymerization involves the linking together of molecules incorporating unsaturated bonds, such as a double or a triple chemical bond between two carbon atoms, and which can be initiated by an ion or a free radical. A free radical is a reactive substance with one unpaired electron which is usually formed by the decomposition of a relatively unstable material called an initiator. Examples of initiators include, but are not limited to, halogen molecules such as chlorine and bromine, and organic peroxides such as benzoyl peroxide and methyl ethyl ketone peroxide. Typically, the free radical can open up an unsaturated bond of a monomer, for example a double bond of a vinyl monomer, and add to it. The resultant molecule remains as a free radical and continues to react with other monomers in the system, until it reacts with another free radical which annihilates each other's growth activity and forms a polymer chain.

The polymer used in the present invention can be
biodegradable. Biodegradable polymers are natural or synthetic polymers that gradually degrade *in vivo* to produce biocompatible or non-toxic byproducts over a period of time (e.g., within days, or months, or years). Disintegration may for instance occur via hydrolysis, may be catalysed by an enzyme and may be assisted by conditions to which the microparticles are exposed in the cell.

The polymers can be biocompatible. As used herein, the term "biocompatible" refers to a material that is capable of interacting with a biological system without causing cytotoxicity, undesired protein or nucleic acid modification or activation of an undesired immune response.

One of the at least three different polymers for manufacturing of a multi-phase microparticle according to a method described herein can be immiscible with at least one of the other polymers. The term "immiscible" as used herein refers to substances that do not mix, i.e. the substances do not form a solution. For example, an organic solvent, such as benzene, and water, or a hydrocarbon, such as soyabean oil, and water, are almost completely immiscible and separate out as two layers upon mixing. Miscibility of the at least three different polymers can be influenced by factors, such as type of and presence or absence of a solvent, total concentration of polymers in the solvent, and temperature. The at least three different polymers according to a method of the present invention can be immiscible with each other in the absence of a solvent. For example, the at least three different polymers can be immiscible with each other when they are mixed together in a solid form or when present in a molten form.

Generally, when the total concentration of the at least three different polymers in solution is low, the at least three different polymers are miscible with each other and can form a homogeneous solution. However, the at least three different polymers can become immiscible with each other in the presence of a solvent. A polymer becomes immiscible with at least one of the other polymers when total concentration of the at least three different polymers reaches a certain concentration in the solvent (e.g. concentration change due to evaporation of solvent), and when reaching this certain concentration separates out as a different phase. The term "phase" refers to a homogenous liquid portion that is present or that can form in a liquid system. A polymer phase comprising a specific polymer in dissolved form can be clearly optically distinguished form other phases, such as by the naked eye. The point at which phase formation takes place is called the cloud point.

The term "cloud point" of a polymer solution refers to the total concentration of the polymers in solution at which one polymer becomes immiscible with the other polymers in the system and forms a separate phase. It can be expressed as a percentage weight of polymer present in the volatile organic solvent by volume of the volatile organic solvent (% w/v). The number of cloud points in a polymer solution is dependent on the number of polymers present. A system making use of two polymers has one cloud point at which the two polymers are immiscible with each other. A system making use of three polymers has two clouds points. The term "first cloud point" of a solution of volatile organic solvent comprising at least three different polymers refers to the lowest total concentration of the polymers in solution at which a first polymer separates out as a phase from the solution. At this first cloud point, one of the at least three different polymers is immiscible with at least one of the other polymers in the system. The term "second cloud point" of a solution comprising at least three different polymers refers to the second lowest total concentration of the polymers in solution at which a second polymer separates out as a phase from the solution. At this second cloud point, the second of the at least three different polymers is immiscible with at least one of the other polymers in the system.

For example, when the total concentration of polymer in the solution increases, such as through removal of the solvent from the solution (e.g. by evaporation) or through addition of polymer into the solution, a first cloud point can be reached in which one of the at least three different polymers is immiscible with at least one of the other polymers in the solvent. The polymer which is immiscible with at least one of the other polymers separates out to form a different phase from the solution, while leaving the other polymers still dissolved in the solvent. When the total concentration of polymer in the solution is further increased, a second cloud point is reached in which a second polymer of the at least three different polymers is immiscible with at least one of the other polymers in the solvent. The second polymer can separate out to form a different phase from the solution. Every time a polymer phase forms, the concentration of the polymer in the phase just formed is measured thus giving us the polymer concentration defining the cloud point.

As an embodiment of using polymers which are not miscible with each other, a polymer system made up of three different types of polymer poly(L-lactic acid) (PLLA), poly(DL-lactic-co-glycolic acid) (PLGA) and poly(caprolactone) (PCL) are used. All three polymers are miscible with each other and are able to dissolve in an organic solvent such as DCM at a total concentration of polymer in solution below the first cloud point. PLGA is not miscible with PLLA and PCL beyond a certain total concentration of polymers in solution i.e. the first cloud point. Therefore, in a solvent extraction process in which the organic solvent is removed from the polymer solution, PLGA is immiscible with PLLA and PCL in the solution, and separates out from the solution as a different phase at the first cloud point, leaving behind PLLA and PCL which are still dissolved in the solvent. As more solvent is removed, total concentration of polymers in solution is further increased, and a second cloud point is reached wherein PLLA is immiscible with PCL in the solution, and PLLA separates out to form a different phase (see also Example 9).

In another embodiment, a polymer system made up of four different types of polymer PLLA, PLGA, polystyrene (PS) and ethylene vinyl alcohol (EVA) are used. All of these four polymers are miscible with each other and are able to dissolve in an organic solvent such as DCM at a total concentration of polymers in solution below the first cloud point. In a solvent extraction process, total concentration of polymers in solution increases and EVA separates out from the solution as a different phase at the first cloud point. As more solvent is removed, total concentration of polymers in the solution is further increased, and PLGA separates out from the solution at the second cloud point. A third cloud point is reached when total concentration of polymers in the solution is further increased, and PS is phase separated with PLLA at the third cloud point (see also Example 14).

The polymers used in accordance with the present invention are (1) PLLA, PLGA and PCL; (2) PLLA, PLGA, PS and EVA; or (3) PLLA, PLGA and EVA.

Reverting to the method of the invention, the method comprises dissolving at least three different polymers in a volatile organic solvent to obtain a first solution. By dissolving in the solvent, the at least three different polymers can form a homogeneous solution. Therefore, a suitable solvent is one that can dissolve each of the at least three polymers. In addition, a suitable solvent is one that is volatile and is immiscible in an aqueous solution. The term "volatile" as used herein refers to a compound that can be readily vaporized at ambient temperature. A measure of the volatility of a substance is its boiling point at one atmosphere. A volatile solvent can have a boiling point at one atmosphere of less than about 100 °C, such as about 75 °C or about 50 °C or about 40 °C or about 30°C. One type or more than one type of solvent can be used.

Suitable solvents that can be used in the present invention includes, but are not limited to, ethyl acetate (EAc), acetone, methyl ethyl ketone (MEK), tetrahydrofuran (THF), chloroform, pentane, benzene, benzyl alcohol, propylene carbonate (PC), carbon tetrachloride, methylene chloride (dichloromethane or DCM) or acetonitrile. In one embodiment of the present invention, methylene chloride (dichloromethane or DCM) is used as organic solvent.

The at least three different polymers can be added together or separately in any order to the solvent. Agitation, for example, by stirring or sonication can be carried out to enhance the rate at which the polymers dissolve in the solvent. Heat energy can optionally be applied to the mixture to increase the dissolve rate of polymers in the solvent.

The method according to the present invention can further comprise addition of a target substance to be encapsulated to the first solution. The term "target substance" refers to a compound which is intended to be delivered or released. The target substance can be encapsulated in the multi-phase microparticle, i.e. the substance can be present on the surface, or enclosed within the multi-phase microparticle. One or more different types of target substances can be added. Depending on the type and strength of interaction between the target substance and polymer, the target substance can be loaded in different regions or layers on the microparticle. The type of interaction between the target substance and the polymer can be physical or chemical in nature. In some embodiments, target substances are loaded in different polymer layers via physical bonding, for example, by any one of hydrophobic forces, hydrogen bonding, Van der Waals interaction, electrostatic forces, or polymer chain entanglement, induced on the polymer chains, such that the target substances are entrapped within the polymer chains. In some embodiments, target substances are loaded in different polymer layers via chemical bonding, for example, by covalent bonding, ionic bonding, or affinity interactions (e.g. ligand/receptor interactions, antibody/antigen interactions, etc.). Therefore, one or more types of target substance can be loaded and localized in different regions or layers of the microparticle by choosing the appropriate type of interaction between the target substance and each of the at least three different polymers, which can be used for controlled release of the target substance in application.

Examples of target substances that can be encapsulated include, but are not limited to, a drug, a protein such as an antibody, and a growth factor, and a nucleic acid. The term "drug" refers to a substance useful for the treatment of or the prevention of a human or an animal disorder or in the regulation of a human or animal physiological condition or metabolic state. Examples of drug include, but are not limited to, antihistamines, e.g. diphenhydramine and chlorphenirmine, and drugs affecting the cardiovascular, renal, hepatic and immune systems, such as antihypertensives, beta blockers, and cholesterol lowering agents; sympathomimetic drugs, such as the catecholamines, e.g. epinephrines; noncatecholamines, e.g. phenylephrine and pseudoephedrine; anti-infective agents, including antibacterial, antiviral and antifungal agents, such as the aminoglycosides, e.g., streptomycin, gentamicin, kanamycin; anti-arthritis drugs, such as narcotic pain relievers; antiinflammatory agents, e.g. indomethacin, dexamethasone and triamcinolone; antitumor agents, e.g. 5-fluorouracil and methotrexate; tranquilizers, such as diazepam; water insoluble, fat-soluble hydrophobic drugs, e.g. steroids such as proqesterone, estrogens, including contraceptives such as ethinyl estradiol and androgens and their analogs, and the fat-soluble vitamins, e.g. vitamins A, D, E and K, and their analogs.

Proteins that can be used as a target substance include, but not limited to, pharmaceutically active ingredients such as hormones, insulins, enzymes, antibodies, and growth factors. The term "growth factors" refers to factors affecting the function of cells such as osteogenic cells, fibroblasts, neural cells, endothelial cells, epithelial cells, keratinocytes, chondrocytes, myocytes, cells from joint ligaments, and cells from the nucleus pulposis. The term "nucleic acid" refers to any nucleic acid in any possible configuration, such as single stranded, double stranded or a combination thereof. Nucleic acids include for instance DNA molecules, RNA molecules, locked nucleic acid molecules (LNA), PNA molecules, analogues of the DNA or RNA generated using nucleotide analogues or using nucleic acid chemistry, DNA-RNA hybrid molecules and tecto-RNA molecules

Other target substances include, for example other pharmaceutical substances, such as a fertilizer, an insecticide or a pesticide, a chemical indicator (such as a pH indicator) or a dye can be encapsulated in the microparticle. As an illustrative example, a drug such as Ibuprofen can be loaded to a triple-layered PLGA/PLLA/EVA microparticle. During the fabrication process, strong affinity between hydrophobic long ethylene chains of EVA and hydrophobic Ibuprofen will drive the drug to be localized in the EVA core.

The first solution is dispersed into an aqueous continuous phase which comprises a surfactant to obtain an emulsion. The term "emulsion" as used herein refers to a disperse system of two or more immiscible liquids. Therefore, emulsifying of one liquid in the other can result in formation of two different phases, in which small droplets of one liquid can be dispersed, i.e. separated and distributed throughout the space, in the other liquid. The small droplets of liquid is called the dispersed phase, while the other liquid, within which the small droplets of liquid is dispersed, is called the continuous phase.

Most emulsions consist of water and oil or fat as immiscible phases. Depending on the composition and ratio of the phases two distribution options exist. In case the aqueous phase, such as water "W" is the continuous phase and the oil "O" is the dispersed phase, the result is an "O/W emulsion" whose basic character is determined by the aqueous phase. If oil "O" is the continuous phase and water "W" the dispersed phase, the result is a "W/O emulsion", wherein the basic character is determined by the oil.

In accordance with this disclosure, an oil-in-oil-in-water (O1/O2/W) emulsion can be used for the manufacture of a ternary-phase double layered microsphere. In this embodiment, three polymers are dissolved in an organic solvent at a concentration between two cloud points forming an O1/O2-emulsion (first emulsion), which makes up the first solution. In other words, O1-phase is formed by one polymer in the organic solvent, and 02-phase is formed by two polymers in the organic solvent, with O1-phase being the dispersed phase and 02-phase being the continuous phase. By dispersing the first solution into an aqueous continuous phase, an O1/O2/W-emulsion (second emulsion) is formed. An illustrative example of an O1/O2/W emulsion is an emulsion formed using PLGA in DCM (O1), PLLA and PCL in DCM (02) and water to which PVA has been added as a surfactant (W).

In another embodiment, an oil-in-oil-in-oil-in-water (O1/O2/O3/W) emulsion can be used to form a triple-layered microparticle. In this embodiment, three polymers are dissolved in an organic solvent at a total polymer solution concentration above the second cloud point. The resultant polymer solution forms an O1/O2/O3 emulsion and is subsequently added to an aqueous continuous phase and emulsified. An illustrative example of an O1/O2/O3/W emulsion is an emulsion formed using PLGA, PLLA and EVA in DCM (O1/O2/O3) and water to which PVA has been added as a surfactant (W).

The principle of microparticle formation as described herein is based upon inducing phase separation of the at least three different polymers dissolved in the organic solvent due to partial extraction of the solvent in a large volume of an external water phase and evaporation of the volatile solvent. Accordingly, concentration of the first polymer of the at least three polymers in the emulsion can be adapted relative to the concentration of the other polymers of the at least three polymers in the emulsion to render the first polymer immiscible with the other polymers of the at least three polymers in the emulsion. As a result of the partial extraction of the solvent, the at least three polymers can coacervate, i.e. separate into two or more immiscible liquid phases, within the emulsion droplets.

The formation of the multi-phase microparticle of the invention will now be illustrated in more detail by means of a polymer solution comprising three polymers as an example. In this embodiment, when the volatile organic solvent is removed from the emulsion droplets dispersed in the aqueous solution, the total concentration of polymers in the emulsion droplets increases until the first cloud point is reached. At this point, small droplets, which contain the polymer with the lowest cloud point and volatile organic solvent, can phase separate, i.e. coacervate forming a first coacervate droplet (or first coacervate phase) within the emulsion droplet. As more solvent is removed from the system, the concentration of the polymer solution increases, and size of the coacervate droplets increases (which can happen as a result of coalescence, i.e. merging of the droplets). The second and third polymers remain miscible within the emulsion droplets. Upon reaching the second cloud point, which in this three polymer system is the last cloud point, the second polymer, which is the one with the second lowest cloud point, and the volatile organic solvent forms a second coacervate droplet (or second coacervate phase) within the emulsion droplet. The second polymer can form a layer on the first coacervate droplet, which lowers the surface energy of the first polymer layer, thus lowering the overall energy of the system. The third polymer that is remaining in the emulsion droplet can then form a separate layer on the second polymer, or can be present as particulate interspersed within the first and/or the second polymer. The third polymer alone does not form microparticles by itself, i.e. separated from the microparticles containing the first and second polymers, as doing so would be thermodynamically unfavorable. The process continues until residual solvent is removed, after which microparticles are formed.

As described herein, microparticles formed from a solution consisting of three different types of polymers can have a triple-layered structure, comprising a core, middle layer and outer layer, with each layer formed from a different polymer. Alternatively, the microparticles formed can also have a double-layered structure, comprising a core and outer layer, with each layer formed from a different polymer, and with interspersed particulates of the third polymer within the first and/or second polymer. The microparticles formed can also have a single layered structure, with interspersed particulates of the second and third polymer within the first polymer.

Likewise, microparticles formed from a solution consisting of four different types of polymers can have a four-layered structure, comprising a core, a second layer, a third layer and an outer layer, with each layer formed from a different polymer. Alternatively, the microparticles formed can also have a three-layered structure, comprising a core, a middle layer and an outer layer, each layer formed from a different polymer, and with interspersed particulates of the fourth polymer within the core, middle and/or outer layer. The microparticles formed can also have a double layered structure, with a first polymer forming a core and a second polymer forming a second layer, and interspersed particulates of the third and fourth polymer within the first and/or second polymer. The microparticles formed can also have a single layered structure, with interspersed particulates of the second, third and fourth polymer within the first polymer.

Accordingly, a multi-phase microparticle comprising discrete regions of different polymer types within the particle can be formed. The configuration of the microparticle, i.e. distribution and size of discrete regions of the different polymer types depends on the rate at which the polymer phases coalesce, and can be controlled by parameters such as concentration of the initial polymer solution, precipitation rate of the polymer system and polymer mass ratio.

The concentration of the initial polymer solution refers to the concentration of the at least three polymers together in the emulsion before evaporation. The concentration of the initial polymer solution can be calculated by dividing the total sum of polymers used by the volume of solvent (expressed as % w/v). It can affect whether the polymers in the emulsified solution can phase separate to form a multi-layered microparticle. To form a multi-phase microparticle, the initial polymer solution concentration comprising at least three different polymers can be lower than the first cloud point, or can be above the first cloud point and below the second cloud point, or can be above the second cloud point of the polymer solution.

For example, to form a ternary phase double layer microparticle, the initial polymer solution concentration can be lower than the first cloud point, or can be between the first and second cloud point of the polymer solution. For example, to form a ternary phase triple layer microparticle, the initial polymer solution concentration can be above the second cloud point of the polymer solution. For example, to form a quaternary phase four layer microparticle, the initial polymer solution concentration is above the third cloud point of the polymer solution.

The choice of initial polymer concentration depends on the viscosity of the polymer solution and the cloud point of polymer in solution. The term "viscosity" as used herein refers to the internal friction of a fluid. It is a measure of the resistance of a fluid under either shear stress or tensile stress deformation, and can be measured using a viscometer. Typically, a polymer with a low molecular weight has a low viscosity, as the polymer chains are shorter and have a lower tendency to entangle with one another. Generally a low molecular weight polymer results in a polymer solution that has a low viscosity when dissolved in a solvent. Without wishing to be bound by theory, it is believed that at a higher polymer concentration, ability of the polymers to move and coalesce with their respective phases decreases due to an increase in viscosity of the polymer solution. This can result in a microparticle structure with no distinctive layers. Accordingly, in order to achieve a multi-phase microparticle, a multiple-polymer system that has a low viscosity and a low cloud point can be used. The low viscosity of the polymer solution can result in greater mobility of the polymers in solution. Likewise, the low cloud point of the polymer solution can result in phase separation of the polymers in solution at a low total polymer concentration, i.e. when the solvent content is high. Therefore, at a low viscosity and a low cloud point of the polymer solution, there is greater mobility of the polymers in solution and the polymer chains can coalesce with their respective phases more readily, thereby forming a multi-phase microparticle configuration.

It will be appreciated that different polymer systems comprising different types of polymers can result in a different viscosity and cloud point required for coalescence of the respective polymer phases to form a multi-phase microparticle configuration. Therefore, in some embodiments, a multi-phase microparticle can also obtained using a multiple-polymer system comprising polymers with a low viscosity and a high cloud point, and in some embodiments, a multi-phase microparticle can also be obtained using a multiple-polymer system comprising polymers with high viscosity and a low cloud point.

In one illustrative embodiment, PLLA, PLGA and PCL having a mass ratio of 3:2:1 are used. When DCM is used as an illustrative solvent and the initial polymer solution concentration used is 2 % w/v (which is less than the first cloud point of 3.75 % w/v in DCM), a double layered microparticle comprising a PLGA core and a PLLA shell is formed. PCL is dispersed as particulates in both the PLGA core and PLLA shell, with a higher concentration of PCL in the PLGA core. When the initial concentration of the polymer solution used is 4 % w/v and 6 % w/v (which are between the first and second cloud point of 3.75 % w/v and 7 % w/v in DCM), a double layered microparticle comprising a PLGA core and a PLLA shell is formed. PCL is dispersed as particulates in both the PLGA core and PLLA shell, with a higher concentration of PCL in the PLLA core. When the initial polymer solution concentration used is 10 % w/v (which is greater than the second cloud point of 7 % w/v in DCM), no distinct layer formation is observed. This can be due to high viscosity of the polymer solution, which results in insufficient time and mobility for the polymers to coalesce to form distinct polymer layers.

The concentration of the initial polymer solution can also influence the size of microparticle formed. Generally, a higher concentration of the initial polymer solution results in formation of a larger size microparticle.

The configuration of the microparticle can be affected by controlling the precipitation rate of the polymer system. The precipitation rate refers to the rate at which solvent is removed from the emulsion. Typically, a lower precipitation rate, i.e. slower rate at which solvent is removed, results in a multi-phase microparticle as there is a greater amount of time and mobility for the polymers to coalesce to form distinct polymer layers.

The precipitation rate is dependent on the initial polymer solution concentration, stirring speed, and ratio of oil phase to aqueous continuous phase. Generally, a lower initial polymer solution concentration results in a lower precipitation rate as the amount of solvent for removal is greater. Without wishing to be bound by theory, it is believed that a lower initial polymer solution concentration provides a longer period of time and greater degree of mobility (as a result of a lower viscosity of the solution) for the polymers to reconfigure themselves according to thermodynamic equilibrium as determined by changes in interfacial energies of the components during solvent extraction.

Generally, a lower stirring speed results in a lower polymer precipitation rate. Stirring speed as used herein can have a range of between about 150 to about 2000 rpm, such as between about 150 to about 1500 rpm, or between about 150 to about 500 rpm. Generally, a lower stirring speed than 150 rpm could result in an insufficient shear force for forming the emulsion droplets. A higher stirring speed can result in a smaller microparticle. A stirring speed exceeding 2000 rpm could result in insufficient coalescence of the respective polymer phases as a result of the high precipitation rate. In one embodiment, a stirring speed of 2000 rpm was used to form a triple-layered multi-phase microparticle which has a smaller diameter of 15 µm (see also Example 15) compared to a triple-layered multi-phase microparticle which has a diameter of about 300 µm at a stirring speed of 400 rpm (see also Example 13).

The ratio of volatile organic solvent to aqueous continuous phase (oil-to-water ratio) refers to the volumetric ratio of volatile organic solvent to water (expressed as v/v). It can be calculated by dividing the volume of volatile organic solvent by the volume of water. Generally, the amount of volatile organic solvent in the polymer system can be controlled to affect the time required for evaporation of the solvent as well as mobility of the polymer phases in solution, which in turn affects the configuration of the microparticles formed. For forming a ternary phase double-walled microparticle, the oil-to-water ratio can be from about 0.1 to about 0.005 v/v, such as from about 0.1 to about 0.03 v/v, or from about 0.1 to about 0.0083 v/v, or from about 0.08 to about 0.03 v/v or from about 0.07 to about 0.04 v/v. For the triple-walled and quadruple-walled microparticles, the ratio of volatile organic solvent to aqueous phase is chosen such that the volatile organic solvent remains soluble in the aqueous continuous phase. As a result of the high precipitation rate, the triple-walled and quadruple-walled structures are kinetically entrapped in non-equilibrium configuration as the polymer chains do not have sufficient time and mobility for reconfiguration.

The polymer mass ratio refers to the mass ratio between different types of polymer. Generally, the polymer phase in solution which has a higher mass forms the outer shell that engulfs the polymer phase that has a lower mass. In the event that the mass of the polymers used is the same for the polymers, the polymer which forms the higher volume can form the shell. Therefore, for a core-shell microparticle, mass ratio of the shell polymer to core polymer can be made large enough to ensure that the shell polymer covers the core polymer. Thickness of each polymer layer of the microparticle can be controlled by varying the polymer mass ratio. Typically, a higher mass of polymer results in a thicker layer of the polymer in the microparticle. Accordingly, the polymer mass ratio can be of any suitable value, which is dependent on the desired configuration of the microparticle. In one illustrative embodiment, a polymer mass ratio for PLLA:PLGA:PCL of 1-3:2-3:1-2 is used.

Different configuration of microparticles such as core-shell inversion can be achieved by varying the polymer mass ratio, which can in turn be dependent on the cloud point and precipitation rate of the polymers. The term "core-shell inversion" as used herein refers to the phenomenon in which a polymer which originally forms the core of a particle reconstructs itself to form a shell over the original shell-forming polymer, but which now forms the core instead, i.e. there is an inversion of the core and shell material.

For example, core-shell inversion can take place through a change in the polymer mass ratio if the polymer solution is prepared above the cloud point of the polymers and at a high precipitation rate. Without wishing to be bound by theory, it is believed that when the first solution comprising at least three different polymers dissolved in a volatile organic solvent is initially poured into an aqueous continuous phase, there is no preferential distribution of a polymer in forming an interface with the surrounding aqueous environment or with other polymers. This preferential distribution of polymer can take place due to presence of hydrophilic or hydrophobic groups on the polymer, whereby presence of the hydrophilic group on the polymer can draw the polymer towards the external aqueous phase, and which can result in the polymer forming the shell of the microparticle. Therefore, when the total concentration of polymer in the solution is prepared about the cloud point of the polymers, and at a high precipitation rate, the polymers are entrapped in their non-equilibrium configuration as there is insufficient time and mobility (due to high concentration of the polymer solution which can result in high viscosity of solution) of the polymers in solution to achieve their equilibrium state. Therefore, by controlling the polymer mass ratio, the original shell material can form the core, and the original core material can form the shell (see for example, Example 13).

A surfactant can be added to stabilize the microparticle containing emulsion. The surfactant thus acts as stabilizer and can include any one of amphoteric surfactant, an anionic surfactant, a cationic surfactant, a nonionic surfactant or mixtures thereof. Depending on the polymers used, the surfactant can influence the size of the microparticle formed.

Addition of an anionic surfactant can render the surface of the microparticle negatively charged. Examples of an anionic surfactant can include, but are not limited to sodium dodecyl sulfate (SDS), sodium pentane sulfonate, dehydrocholic acid, glycolithocholic acid ethyl ester, ammonium lauryl sulfate and other alkyl sulfate salts, sodium laurcth sulfate, alkyl benzene sulfonate, soaps, fatty acid salts or mixtures thereof.

Addition of a cationic surfactant can render the surface of the microparticle positively charged. Examples of a cationic surfactant can include, but are not limited to cetyl trimethylammonium bromide (CTAB), dodecylethyldimethylammonium bromide (D12EDMAB), didodecyl ammonium bromide (DMAB), cetylpyridinium chloride (CPC), polyethoxylated tallow amine (POEA), hexadecyltrimethylammonium p-toluenesulfonate, benzalkonium chloride (BAC), benzethonium chloride (BZT) and mixtures thereof.

Examples of a nonionic surfactant can include, but are not limited to poloaxamers, alkyl poly(ethylene oxide), diethylene glycol monohexyl ether, polyvinyl alcohol (PVA), copolymers of poly(ethylene oxide) and poly(propylene oxide), hexaethylene glycol monohexadecyl ether, alkyl polyglucosides, digitonin, ethylene glycol monodecyl ether, cocamide MEA, cocamide DEA, cocamide TEA, fatty alcohols or mixtures thereof. In one example, polyvinyl alcohol (PVA) has been used to stabilize the microsphere emulsion.

Poloaxamers, such as F127, are difunctional block copolymer surfactants terminating in primary hydroxyl groups. They are composed of a central hydrophobic chain of polyoxypropylene (poly(propylene oxide)) flanked by two hydrophilic chains of polyoxyethylene (poly(ethylene oxide)). Because the lengths of the polymer blocks can be customized, many different poloxamers exist having slightly different properties. For the generic term "poloxamer", these copolymers are commonly named with the letter "P" (for poloxamer) followed by three digits, the first two digits x 100 give the approximate molecular mass of the polyoxypropylene core, and the last digit x 10 gives the percentage polyoxyethylene content (e.g., P407 = Poloxamer with a polyoxypropylene molecular mass of 4,000 g/mol and a 70% polyoxyethylene content). For the Pluronic tradename, coding of these copolymers starts with a letter to define it's physical form at room temperature (L = liquid, P = paste, F = flake (solid)) followed by two or three digits, the first digit(s) refer to the molecular mass of the polyoxypropylene core (determined from BASF's Pluronic grid) and the last digit x 10 gives the percentage polyoxyethylene content (e.g., F127 = Pluronic with a polyoxypropylene molecular mass of 4,000 g/mol and a 70% polyoxyethylene content). In one example, poloxamer 407 (P407) (= Pluronic F127) or F-188 or L-63 or mixtures thereof can be used.

Examples of an amphoteric surfactant can include, but are not limited to dodecyl betaine, sodium 2,3-dimercaptopropanesulfonate monohydrate, dodecyl dimethylamine oxide, cocamidopropyl betaine, 3-[N,N-dimethyl(3-palmitoylaminopropyl)ammonio]-propanesulfonate, coco ampho glycinate and mixtures thereof.

Reverting to the last step of the method of the invention, this final emulsion is mixed at a suitable temperature or under vacuum to allow evaporation of the organic solvent. After evaporation of the solvent, the microparticles can be centrifuged and washed before storage.

In a second aspect, the present invention refers to a multi-layered microparticle obtained by a method of the present invention. For a multi-layered microparticle comprising a target substance, the multi-layered microparticle can be degraded at a site of intended usage to release the target substance. The term "degrade" as used herein refers to breaking down of the multi-layered microparticle to smaller molecules. Degradation can take place under certain conditions, such as temperature, abrasion, pH, ionic strength, electrical voltage, current effects, radiation and biological means. In some embodiments, degradation of the microparticle can take place over a time period ranging from a few seconds to a few days or months. The time period required for the microparticle to degrade can be dependent on a few parameters, for example, constituent of the microparticle, such as type of polymer and target substance used, size of the microparticle, temperature, pH and pressure.

In a third aspect, the present invention refers to a pharmaceutical composition comprising a multi-layered microparticle obtained by a method of the present invention. In some embodiments, the composition can be poured or injected into a mold having a desired shape, and then hardened to form a matrix having microparticles dispersed therein. The polymers can degrade, leaving only the target substance. Therefore, a sustained release of the target substance such as fertilizer or pesticide can be achieved along with degradation of the polymers. In some embodiments, the composition is adapted to be deliverable to a site, such as a defect site, in an animal or a human body. The composition can be injected directly into a site, such as a defect site, in a patient, where the polymer can harden into a matrix having microparticles dispersed therein. The polymer can be biodegradable. Therefore, a sustained release of the target substance, such as drugs, can be achieved along with degradation of the polymers.

Other embodiments are within the following claims and non- limiting examples.

### EXPERIMENTAL SECTION

### Example 1: Materials

Poly(L-lactic acid) (PLLA, intrinsic viscosity/IV: 2.38, Bio Invigor), poly(DL-lactic-co-glycolic acid 50:50) (PLGA, IV: 1.18, Bio Invigor), poly(caprolactone) (PCL, Aldrich) and poly(vinyl alcohol) (PVA, MW 30 - 70 kDa, Sigma-Aldrich) were used without further purification. Properties of the polymers used in this study are listed in Table 1. High-Performance Liquid Chromatography (HPLC) grade Dichloromethane (DCM) and Tetrahydrofuran (THF) (Tedia Company Inc.) were used as solvents, as received.

**Table 1: Polymers used in the study**

| Polymer | Intrinsic viscosity (dlg⁻¹) | Mₙ(gmol⁻¹)* |
|---|---|---|
| PLLA | 2.38 | 1.64 x 10⁵ |
| PLGA | 1.18 | 5 x 10⁴ |
| PCL | - | 10.7 x 10⁴ |

| | | |
|---|---|---|
| *Number-average molecular weight as determined by gel permeation chromatography (GPC). | | |

### Example 2: Polymer cloud point determination

To determine the cloud point of a polymer, i.e. polymer solution concentration at which one polymer became immiscible with the other two polymers, a mass of 0.3 g of the three polymers were weighed according to a mass ratio of 3:2:1 (PLLA: PLGA: PCL). To prepare a 2 % (w/v) homogenous polymer solution, PLLA (0.15 g), PLGA (0.1 g) and PCL (0.05 g) were dissolved in 15 mL of DCM. The ternary-polymer solution was then transferred to a 20 mL graduated cylinder and allowed to sit undisturbed in a fume hood at room temperature. When two distinct phases became apparent in the solution due to polymer phase separation as shown in Figure 20A, the volume of the solution was recorded by reading off the scale on the graduated cylinder. Phase separation of PLGA was detected when a distinct yellowish liquid phase was formed. To determine the polymer i.e. PLGA, PLLA or PCL in each phase, the polymer phase that is extracted using a syringe can be analyzed by Fourier Transform Infrared Spectroscopy (FTIR).

To further confirm if the polymer solution is prepared above the cloud point of all the polymers, the solution is centrifuged at 3500 rpm for 10 minutes. If three distinct layers are observed, this implies that all three polymers in the solution become immiscible above the cloud point. This is shown in Figure 20B, which is a photograph showing PLGA/PLLA/EVA solution at 7.5 % w/v. The cloud point of the polymer system is 5 % w/v. Similarly, four distinct layers can be observed for a four polymer system as shown in Figure 20C, which is a photograph showing PLGA/PLLA/PS/EVA solution at 13 %w/v. The cloud point of the polymer system is 12 %w/v.

### Example 3: Fabrication of microparticles

The PLLA/PLGA/PCL composite microparticles were prepared using an (O/W) emulsion solvent evaporation method. The three polymers were first dissolved in DCM. The resultant polymer solution was added to a PVA aqueous solution of 0.5% (w/v) and emulsified using an overhead stirrer (Calframo BDC1850-220). Evaporation of DCM will give rise to phase separation of PLLA, PLGA and PCL, to yield ternary-phase composite microparticles. Finally, the microparticles were filtered, rinsed with de-ionized water, lyophilized and stored in a desiccator for further tests.

Microparticles with different configurations were prepared in the same manner by altering the starting ternary-polymer solution concentration, stirring speed, oil-to-water ratio and polymer mass ratio. Table 2 summarizes the process parameters used in the solvent evaporation method to fabricate different configurations of ternary-phase microparticles. A reference ternary-phase microparticle (Particle R) was fabricated for subsequent comparison with other microparticles.

**Table 2: Parameters used to fabricate the PLLA/PLGA/PCL composite microparticles**

| Description | Polymer solution concentration (% w/v) | Stirring speed (rpm) | Oil-to-water ratio | Polymer mass ratio | | |
|---|---|---|---|---|---|---|
| | | | | PLLA / PLGA / PCL | | |
| Ternary-phase reference microparticle (R) | 6 | 300 | 0.02 | 0.15g | 0.1g | 0.05g |
| | | | | 3 : | 2 : | 1 |
| Effect of starting polymer solution concentration | 2 | 300 | 0.02 | 0.15g | 0.1g | 0.05g |
| | 4 | | | | | |
| | 6 | | | 3 : | 2 : | 1 |
| | 10 | | | | | |
| Effect of stirring speed | 6 | 150 | 0.02 | 0.15g | 0.1g | 0.05g |
| | | 300 | | | | |
| | | 400 | | 3 : | 2 : | 1 |
| | | 500 | | | | |
| Effect of oil-to-water ratio | 6 | 300 | 0.1 | 0.15g | 0.1g | 0.05g |
| | | | 0.03 | | | |
| | | | 0.02 | 3 : | 2 : | 1 |
| | | | 0.0125 | | | |
| | | | 0.0083 | | | |
| Effect of polymer mass ratio | 6 | 300 | 0.02 | 0.1g | 0.15g | 0.05g |
| | | | | 2 : | 3 : | 1 |
| | | | | 0.05g | 0.15g | 0.05g |
| | | | | 1 : | 3 : | 1 |
| | | | | 0.15g | 0.1g | 0.1g |
| | | | | 3 : | 2 : | 2 |

### Example 4: Characterization using Scanning Electron Microscope (SEM)

Surface and internal morphologies of microparticles were analysed using scanning electron microscope (SEM). The SEM used was JEOL JEM-6360A, which was operated at a voltage of 5 kV. Before analysis, the samples were first mounted onto metal stubs and cross-sectioned approximately at the centreline with a razor blade. Samples were then coated with gold using a sputter coater model SPI-Module. The formation efficiency of the complete engulfing structure was quantified by calculating the percentage of microparticles, embedded in a polyacylic resin, which were double-walled compared to the total number of microparticles in a field of view under SEM.

### Example 5: Microparticle configuration or polymer distribution study using dissolution method

The dissolution method based on the solubility differences of the polymers in THF (PLGA and PCL are soluble in THF, but not PLLA), was used to determine the final configuration or polymer distribution within the ternary-polymer microparticles.

The cross-sectioned composite particles were first immersed in THF, without agitation, for 2 days to dissolve the PLGA and PCL contents. The cross-sectioned particles were then collected for SEM analysis.

### Example 6: Microparticle configuration or polymer distribution study using Raman mapping

Raman mapping was used as verification of the final particle configuration obtained from the dissolution method illustrated in Example 5. Composite microparticles that have been pre-sectioned were placed under the microscope objective with laser power up to circa 20 mW. Raman point-by-point mapping measurements were then performed on the area of 300 µm x 200 µm with a step size of 5 µm in both the x and y directions using a Raman microscope (InVia Reflex, Renishaw) equipped with near infrared enhanced deep-depleted thermoelectrically Peltier cooled CCD array detector (576 x 384 pixels) and a high grade Leica microscope. The sample was irradiated with a 785 nm near infrared diode laser and a 50 x objective lens was used to collect the backscattered light. Measurement scans were collected using a static 1800 groove per mm dispersive grating in a spectral window from 300 to 1900 cm⁻¹ and acquisition time for each spectrum was around 35 seconds. Spectral preprocessing that included spike removal due to cosmic rays were then carried out first before the Raman mapping data was further analyzed using the band-target entropy minimization (BTEM) algorithm. BTEM algorithm was developed in order to reconstruct the pure component. When all normalized pure component spectra of all underlying constituents were reconstructed, the relative contributions of each measured point of these signals were calculated by projecting them back onto the baseline-corrected and normalized data set. The spatial distribution or polymer distribution of each underlying constituents (i.e. PLLA, PLGA and PCL) was then generated.

### Example 7: Microparticle configuration or polymer distribution study using Differential Scanning Calorimetry (DSC)

Thermal analysis of the microparticles was performed using a differential scanning colorimeter (DSC) (DSC-Q10, TA Instruments). To avoid oxidative degradation, the sample and reference pans were purged with nitrogen at a constant flow rate of 48 mL/min. Approximately 5 mg of each sample was heated from -50 °C to 200 °C at a scan rate of 5 °C/min.

### Example 8: Fabrication of ternary-phase reference microparticles (Particle R)

A reference set of ternary-phase microparticles (R) was fabricated for comparison with the microparticles obtained using the process parameters as listed in Table 2. Figure 1 shows the SEM micrographs for R. Figure 1A is a micrograph showing the external morphology of a R particle. Figure 1B is a micrograph showing a cross-section of a R particle. Figure 1C is a micrograph which shows a close-up view of the internal morphology of a R particle. Figure 1D is a micrograph showing a cross-section of R particles embedded in a polyacrylic resin.

Microparticle configuration or polymer distribution can be categorized in the following ways, i.e. a) size of microparticle, b) relative size and position of shell, core and particulate, and c) distribution of particulate in shell and/or core.

Using the micrographs, the R particle was found to have a size range of about 300-400 µm. As shown in Figure 1A, the R particle is spherical with a smooth exterior surface. A complete engulfing structure was observed as shown in Figure 1B. It was found that the shell of the microparticle was less dense as compared to the core as shown in Figure 1C. Figure 1D shows a cross-section of the microparticles embedded within a polyacrylic resin, which confirms the observation that the microparticles have a complete engulfing structure. Formation efficiency of this complete engulfing structure was estimated to be about 100%.

Figure 2 are SEM micrographs showing cross-sectional views of a R particle after dissolution in THF. A hollow core with a surrounding porous shell can be seen from Figure 2A. As PLLA is insoluble in THF, and both PCL and PLGA are soluble in THF, therefore the shell was identified as PLLA. Figure 2B is a close-up view of the porous shell. Size of the pores was found to be in the range of about 5 to 10 µm.

The core-shell configuration was further verified using Raman mapping. Raman measurement results are shown in Figure 3. The results confirmed the dissolution results, in that the shell and core were PLLA and PLGA-abundant regions respectively, and that PCL was dispersed in the PLLA shell. Therefore, with reference to Figure 2B, size of PCL particulates can be said to be in the range of about 5 to 10 µm, which is consistent with the dissolution results.

Therefore, as observed using SEM, dissolution method, and Raman mapping, the R particles formed comprise a PLGA core, having a PLLA shell that is impregnated with PCL particulates. For illustration purposes, composites of PLLA/PLGA were fabricated. Figure 4 are SEM micrographs showing PLLA/PLGA particles. In the absence of PCL, a dense PLLA shell was observed from double-walled dual-phase PLLA/PLGA microspheres.

DSC thermograms of the R particle showed two glass transition temperatures, which correspond to the glass transition temperature of PLLA and PLGA respectively, and two melting temperatures, which correspond to the melting temperature of PLLA and PCL respectively. Thermal properties of the pure polymers and Particle R obtained from DSC thermograms are shown in Table 3.

**Table 3: Thermal properties of pure polymers and composite microparticle obtained from DSC thermograms**

| Polymers | Glass transition temperature, Tg (°C) | | Melting temperature, Tm (°C) | |
|---|---|---|---|---|
| PLGA | 43 | | - | |
| PLLA | 74 | | 180 | |
| PCL | - | | 60 | |
| PLLA/PLGA/PCL | PLLA: 63 | PLGA: 45 | PLLA: 176 | PCL: 52 |

The existence of two glass transition temperatures and two melting temperatures from the DSC thermograms, which are representative of the original polymers, implies that the polymers were phase-separated and formed an immiscible blend, thereby resulting in the observed ternary-phase microparticles.

### Example 9: Effect of starting polymer solution concentration on particle configuration

Using cloud point measurements of PLLA/PLGA/PCL, a first cloud point of 3.75% (w/v) at which PLGA began to phase separate from PCL/PLLA, and a second cloud point of 7% (w/v) at which PCL and PLLA began to phase separate, were observed. As such, using these values, the initial polymer solution concentrations were prepared at 2 % (w/v) (below first cloud point), 4 % (w/v), 6 % (w/v) (between first and second cloud point), and 10 % (w/v) (above both cloud points).

Figure 5 are SEM micrographs of PLLA/PLGA/PCL microparticles prepared using various polymer solution concentration, i.e. (A) 2 % (w/v), (B) 4 % (w/v), (C) 6 % (w/v) and (D) 10 % (w/v).

As shown in Figure 5A, when the initial polymer solution concentration was at 2 % (w/v), a complete engulfing configuration was observed, wherein the core appeared to be less dense as compared to the shell. The porous morphology of the core indicates more PCL being dispersed in the PLGA core for 2 % (w/v). When the initial polymer solution concentration was increased to 4 % (w/v) and 6 % (w/v) as shown in Figure 5B and 5C respectively, configuration of microparticles remained double-walled, and which is similar to that of reference R. However, at an initial polymer solution of 10 % (w/v) as shown in Figure 5D, no engulfing structure was observed. On the contrary, incomplete coalescence of the polymers with their respective phases was observed. It was also observed that an increase in polymer solution concentration increases size of the microparticles formed.

Therefore, from the results obtained, it can be concluded that in order to achieve a double-walled configuration of the microparticles, a low initial polymer solution concentration is required. A suitable initial polymer solution concentration can be determined using cloud point measurements. In addition, a larger size of microparticle is obtained for a higher polymer solution concentration, which can be due to larger emulsion droplets as a result of the more concentrated solution.

Without wishing to be bound by theory, and using the polymer system of the reference R as example, it is believed that when the polymer solution having an initial polymer concentration of 6% (w/v) was poured into the PVA aqueous solution, PLGA phase separated as small droplets containing DCM and polymer (known as coacervate phase) within the emulsion droplets as the cloud point of 3.75% (w/v) at which PLGA was phase-separated from PCL/PLLA was reached. These droplets start to coalesce, becoming larger in size, while PLLA and PCL were still miscible within the emulsion droplets.

Upon reaching the second cloud point at 7% (w/v), PCL and PLLA began to phase separate. During solvent evaporation, PLGA coacervate droplets tend to migrate towards the inner core. This is because DCM-rich PLGA coacervate droplets were more hydrophobic than PLLA-DCM phase due to a higher degree of interaction between DCM and PLGA, as shown by the higher solubility of PLGA in DCM. On the other hand, PLLA coacervate droplets had greater affinity for the surrounding aqueous phase. PCL is shown to exist as particulates in the PLLA layer, which could be a result of the following. Firstly, intermixing of PCL with the larger PLGA coacervate droplets could be difficult, and given its limited mobility in a dispersing PLLA phase, PCL also migrate to the outer region of the emulsion droplet together with PLLA. Secondly, the faster solidification of PLLA also means that the time provided for PCL to further coalesce and migrate is reduced. Thirdly, the high molecular weight (high viscosity) of PLLA and PCL, and lower PCL content in the system (i.e. mass ratio of 3 PLLA: 1 PCL) may also hinder the coalescence and migration of PCL, leading to smaller PCL particulates embedded in the PLLA phase. Further removal of solvent finally caused PLLA to precipitate, forming a shell and trapping the PCL particulates inside, with PLGA forming the inner core.

Therefore, it is believed that one of the main factors in determining the polymer configuration is chain mobility of the polymers in solution.

Applying this concept on the polymer solution having an initial polymer concentration of 2% (w/v), the polymers inside the emulsion droplets remained miscible until phase separation of PLGA ensued at the first cloud point of 3.75% (w/v). The high molecular weight, which leads to a high viscosity, of PLLA and PCL makes phase separation of PLGA from PCL/PLLA phase more difficult. The surface tensions of PLGA, PLLA and PCL are 37.7 mJ/m², 36.1 mJ/m² and 37.5 mJ/m² respectively, while the surface tensions of the polymer solutions can be predicted from a volume-weighted average of the polymer and solvent. The effective interfacial energies and thus the predicted thermodynamic configuration will change as the solvent is evaporated. As calculated using the Good and Girifalco equation (see for example, Girifalco, L.A.; Good, R.J. J. Phys. Chem. 1957, 61 904), the surface tension of PLGA and PCL are more similar compared to PLLA and PCL. Therefore there is lower interfacial energy between PLGA and PCL. As such, the homogeneous dispersion of the small PLGA coacervate droplets as well as lower interfacial energy between PLGA and PCL enhanced the interaction and intermixing between PLGA and PCL coacervate droplets when the second cloud point was reached. This will give rise to more PCL being dispersed in the PLGA core as shown in Figure 5A. In addition, a lower precipitation rate can facilitate the thermodynamically driven configuration of polymers towards thermodynamic equilibrium configurations, which can be affected by the interfacial energies between the PLGA and PCL coacervate droplets.

As a further example, when the starting polymer solution has a concentration of 4% (w/v) and 6% (w/v), which are between the two cloud points, the same microparticle configuration, i.e. a PLGA core, and a PLLA shell with interspersed PCL particulates, is obtained.

When the polymer concentration is at 10% (w/v), the high polymer concentration results in an increased viscosity, which reduces the time allowed for the polymers to move into and coalesce with their respective phases. As such, no engulfing structure was observed as shown in Figure 5D.

### Example 10: Effect of stirring speed on particle configuration

Figure 6 are SEM micrographs of PLLA/PLGA/PCL microparticles prepared with a stirring speed of (A) 150 rpm, (B) 300 rpm, (C) 400 rpm and (D) 500 rpm. It was observed that changes in stirring speed did not affect the final configuration i.e. distribution of polymers in the microparticles. As shown from the figures, the double-wall structure is retained in the microparticles. It was observed that at the lowest stirring speed of 150 rpm, PCL particulates in the PLLA shell were the largest in size as shown in Figure 6A. This could be due to a decrease in polymer precipitation rate at lowering stirring speeds, thereby resulting in PCL coacervate droplets having more time to coalesce inside the PLLA matrix before solidification of PLLA takes place. This observation was confirmed using the THF dissolution test, which showed larger pores in the PLLA shell as shown in Figure 7.

It was also observed that an increase in stirring speed reduced the size of the microparticles formed. This could be due to a higher stirring speed providing finer emulsion droplets through strong shear forces, thereby resulting in smaller particle sizes.

### Example 11: Effect of oil-to-water ratio on particle configuration

Effect of oil-to-water ratio on the microparticle structure was studied. Figure 8 arc SEM micrographs PLLA/PLGA/PCL microparticles prepared with an oil-to-water ratio of (A) 0.1, (B) 0.03, (C) 0.02, (D) 0.0125 and (E) 0.0083.

For the lowest volume of PVA solution (oil-to-water ratio = 0.1), the PLGA coalescence phase was not located at the centre of the particle but was observed to migrate away from the centre, as shown in Figure 8A. In addition, the PCL particulates in the PLLA shell were observed to be larger in size when a lower volume of PVA aqueous solution was used, as can be seen from Figure 8A and 8B. However, Figure 8C, 8D and 8E shows that further increasing the volume of the continuous phase of PVA did not result in any further effect on the double-walled configuration of microparticles.

Without wishing to be bound by theory, it is believed that chain mobility of the polymers in solution is affected by the initial oil-to-water ratio of the polymeric system, which in turn affects the polymer configuration. For example, during the solvent removal process, DCM must first diffuse into the aqueous phase and then evaporate at the water/air interface. (Diffusion of DCM takes place as a result of concentration gradient.) DCM is only slightly soluble in water, with a solubility of about 2% (v/v). An initial oil-to-water ratio of 0.02, which translates into 5 mL of DCM to 250 mL of the PVA aqueous solution, was considered to be a critical point. The critical point is derived based on the amount of DCM used, which is in turn depending on the polymer solution concentration and total polymer mass, and the solubility of DCM in water, and is different for different solvent used. At oil-to-water ratios greater than this critical point, the water content is relatively low. This results in a slow diffusion of DCM into the aqueous phase, thereby causing a slow precipitation of the polymers. This also applies to PCL coacervate droplets, which were observed to be larger in size when a lower volume of PVA aqueous solution was used, since they have more time to coalesce.

At a later stage of the solvent removal process, the small amount of remaining DCM no longer made PLGA coacervate droplets more hydrophobic than PLLA. As a result, PLGA expressed its higher hydrophilicity by migrating towards the external aqueous phase when the precipitation rate was slow enough. This is shown in Figure 8A in which the PLGA coalescence phase was not located at the centre of the particle but was observed to migrate away from the centre. Beyond the critical oil-to-water ratio of 0.02, the amount of aqueous solution did not seem to have any effect on the final configuration of particles. It is possible that, when the aqueous volume is too large, DCM diffuses into the water without any delay. Therefore, the solvent extraction rate was unaffected by the oil-to-water ratio beyond a critical point.

### Example 12: Effect of polymer mass ratio on particles configuration

Figure 9 are SEM micrographs of microspheres prepared using PLLA: PLGA: PCL mass ratio of (A) 2: 3: 1, (C) 1: 3: 1, and (E) 1: 3: 1. Figure 9B, 9D and 9F are the respective corresponding cross-sectional views of Figure 9A, 9C and 9E after dissolution with THF.

From the figures, it is observed that a change in the mass ratio of polymers in the starting solution results in a change in the resultant size of the embedded particulates and dimensions of the core and shell structure. For example, when comparing between Figure 1B, which uses a PLLA: PLGA: PCL mass ratio of 3: 2: 1 for the reference R particle, with Figure 9A, which uses a PLLA: PLGA: PCL mass ratio of 2: 3: 1, a core of larger diameter and shell of smaller thickness was observed. With a lower PLLA to PLGA ratio, a PLGA core of larger diameter and a PLLA shell of smaller thickness were observed. Ratio of inner core volume to outer shell volume became even higher when PLLA: PLGA: PCL mass ratio is changed to 1: 3: 1 as can be seen in Figure 9C, since the PLLA (shell): PLGA (core) mass ratio is changed to 1: 3. Figure 9E and 9F, which uses a PLLA: PLGA: PCL mass ratio of 1: 3: 1 show that an increase in the amount of PCL resulted in the formation of larger PCL particulates in the PLLA shell.

This observation is confirmed using the SEM micrographs of double-walled composite microparticles after dissolution with THF in Figure 9B, 9D and 9F, the shell and core composition were identified as PLLA and PLGA respectively, and the shell was impregnated with PCL particulates. Furthermore, core-shell inversion was not observed for the different polymer mass ratios used. This is surprising as it has been reported by other groups that core-shell inversion can be obtained by varying the polymer ratio (see, for example, Leach K et al, Journal of Microencapsulation 1999, 16, 153-67, Tan EC et al, Journal of Colloid and Interface Science 2005, 291, 135-43, and Matsumoto A et al, Journal of Controlled Release 1997, 48, 19-27). In the studies carried out by other groups, when a binary-polymer solution was initially poured into the PVA aqueous solution, the surface tension of each of the polymer phases is about the same due to similar DCM content. Therefore, the higher mass polymer usually forms the outer shell that engulfs the polymer phase with lower mass. This phenomenon occurs when the precipitation rate is fast enough to kinetically trap the non-equilibrium configuration in order for the core-shell inversion to take place. As such, the inventors of the present invention believed that a core-shell inversion occurs only when the polymer solution is prepared above the cloud point at which an oil-in-oil emulsion is created by sonication with a well-controlled precipitation rate.

Therefore, with a considerably low starting polymer concentration at 6% (w/v) as used in the examples, the inventors believe that there is sufficient time for the polymer phases to reconfigure themselves according to the thermodynamic equilibrium dictated by their interfacial energies. As such, no core-shell inversion was observed. Instead, dimension of the shell and core of the microparticle can be fine tuned accordingly by changing the mass ratio of different polymers.

### Example 13: Formation of a triple-layered microparticle and its layer inverted form

Table 4 summarises the settings used for forming a PLGA/PLLA/EVA triple-walled microparticle (EVA core, PLLA middle layer and PLGA outer layer), and its layer inverted form i.e. a PLLA/PLGA/EVA tripled walled microparticle (EVA core, PLGA middle layer and PLGA outer layer).

**Table 4: Settings used to form triple-walled PLGA/PLLA/EVA microparticles**

| Description | Polymer solution concentration (% w/v) | Stirring speed (rpm) | Oil-to-water ratio | Polymer mass ratio | | |
|---|---|---|---|---|---|---|
| | | | | PLLA / PLGA / EVA | | |
| | | | | | | |
| PLGA/PLLA/EVA (shell to core) microparticles | 7.5 (second cloud | 400 | ∼0.013 (350 mL PVA | 0.2g | 0.1g | 0.05g |
| | | | | 4 : | 2 : | 1 |
| | point is 5% w/v) | | solution) | | | |
| PLLA/PLGA/EVA (shell to core) microparticles | 7.5 (cloud point of all the polymer is 5%w/v) | 400 | ∼0.013 (350mL PVA solution) | 0.1g | 0.2g | 0.05g |
| | | | | 2 : | 4 : | 1 |

Generally, the microparticles are prepared using an emulsion solvent evaporation method. The three polymers are first dissolved in DCM. After which, the resultant polymer solution is first ultra-sonicated for 1 min and was subsequently added to a PVA aqueous solution (0.5% (w/v)) and emulsified using an overhead stirrer (Calframo BDC1850-220) at room temperature (25°C). Evaporation of DCM results in the phase separation of PLLA, PLGA and EVA, and triple-walled microparticles are obtained. The microparticles were filtered, rinsed with de-ionized water, lyophilized, and stored in a desiccator.

Figure 10A is a SEM micrograph of a triple-walled (TW) microsphere of ternary phase poly(DL-lactic-co-glycolic acid/poly(L-lactic acid)/ethylene vinyl acetate (PLGA/PLLA/EVA), corresponding to the outermost shell, middle layer and core respectively. Figure 10B shows pure component Raman spectra estimates and their associated score images obtained via BTEM from the ternary phase PLGA/PLLA/EVA triple-walled composite microparticle. Figure 10C is a SEM micrograph of a triple-walled microsphere of PLGA/PLLA/EVA, in which the PLGA shell has a larger thickness. Figure 10D shows a SEM micrograph of a layer-inverted ternary phase PLLA/PLGA/EVA microsphere, in which the outermost shell, middle layer, and core are PLLA, PLGA, and EVA respectively. Figure 10E shows pure component Raman spectra estimates and their associated score images obtained via BTEM from the ternary phase PLLA/PLGA/EVA triple-walled composite microparticle.

### Example 14: Formation of a four-layered microparticle

Table 5 summarises the settings used for forming a PLGA/PLLA/PS/EVA four layer microparticle (EVA core, PS second layer, PLLA third layer and PLGA outer layer).

**Table 5: Settings used in formation of a PLGA/PLLA/PS/EVA four layer microparticle**

| Polymer solution concentration [% w/v] | Polymer mass ratio | | | |
|---|---|---|---|---|
| 13 | PLGA | PLLA | EVA | PS |
| (cloud point of all the polymer is 12%w/v) | 0.4g | 0.2g | 0.1g | 0.04g |
| | 10 : | 5 : | 2.5 : | 1 |

The polymers were first dissolved in DCM. After which, the resultant polymer solution was ultra-sonicated for 1 min and is subsequently added to water containing a PCA surfactant (0.5% (w/v)) with oil-to-water ratio of 0.013 and emulsified at 400 rpm using an overhead stirrer (Calframo BDC1850-220) at room temperature (25 °C). The evaporation of DCM results in the phase separation of the polymers, yielding multi-phase composite microparticles. Finally, the microparticles were centrifuged, rinsed with de-ionized water, lyophilized, and stored in a desiccator.

Figure 11A is a SEM micrograph of a four-layered poly(DL-lactic-co-glycolic acid/poly(L-lactic acid)/poly styrene/ethylene vinyl acetate (PLGA/PLLA/PS/EVA) microparticle. Shell to core configuration: PLGA, PLLA, PS, EVA. Figure 11B shows pure component Raman spectra estimates and their associated score images obtained via BTEM from the PLGA/PLLA/PS/EVA four-layered composite microparticle.

### Example 15: Formation of a smaller diameter three-layered microparticle

Ternary phase triple walled PLGA/PLLA/EVA (core EVA, middle layer PLLA and outer layer PLGA) with a smaller diameter of about 15 µm was formed using a higher stirring speed of 2000 rpm and higher surfactant content of 2 % w/v. The other process parameters were the same as that used for PLGA/PLLA/EVA microparticles in Example 13. As a result of finer emulsion droplets formed from the stronger shear forces resulting, smaller particle sizes were formed.

Figure 12A is a SEM micrograph of a triple-walled PLGA/PLLA/EVA microparticle, corresponding to the outermost shell, middle layer and core respectively, having a size of about 15 µm. Figure 12B shows pure component Raman spectra estimates and their associated score images obtained via BTEM from the PLGA/PLLA/EVA triple-walled composite microparticle.

### Example 15: Localisation of target substance in microparticle

For the fabrication of Ibuprofen-loaded triple-layered PLGA/PLLA/EVA microparticles, Ibuprofen is first added to the polymer solution and the resulting solution is subsequently ultra-sonicated for 1 min to achieve complete homogenization. This solution will then be poured into the PVA aqueous solution under stirring. The interaction between the drugs and polymers (polymer-drug interaction) is the main driving force for the distribution of drugs. The higher the affinity between the drug and polymer, the stronger is the tendency for the drug to be loaded into the polymer. During the fabrication process, strong affinity between hydrophobic long ethylene chains of EVA and hydrophobic Ibuprofen drove the drug to be localized in the EVA core. This drug-polymer affinity allows for different drugs, based on their hydrophilic-hydrophobic nature, to be localized in different polymers, thus resulting in specific layer localization of drugs.

Figure 13A is a SEM micrograph of a ternary phase PLLA/PLGA/PCL dual-walled composite microparticle. Ibuprofen (hydrophobic) is localized in the PLLA shell, impregnated with PCL particulates; Metoclopramide HCl (hydrophilic) is localized in the PLGA core. Figure 13B shows pure component Raman spectra estimates and their associated score images obtained via BTEM from the microparticle.

Figure 14A is a SEM micrograph of a ternary phase PLGA/PLLA/EVA triple-walled composite microparticle. The outermost shell, middle layer, and core are PLGA, PLLA, and EVA abundant regions respectively. Ibuprofen (hydrophobic) is localized in the EVA core. Figure 14B shows pure component Raman spectra estimates and their associated score images obtained via BTEM from the microparticle. Figure 14C is a SEM micrograph of a ternary phase PLGA/PLLA/EVA triple-walled composite microparticle. The outermost shell, middle layer, and core are PLGA, PLLA, and EVA abundant regions, respectively. Metoclopramide HCl (hydrophilic) is localized in the PLGA shell. Figure 14D shows pure component Raman spectra estimates and their associated score images obtained via BTEM from the microparticle.

Figure 15 is a graph showing drug release profiles of ternary phase dual-walled PLLA/PLGA/PCL microparticles with mass ratios of A) 6:2:1, B) 3:2:1, and C) 3:2:2. The shell and core are PLLA impregnated with PCL particulates, and PLGA abundant regions respectively. Metoclopramide HCl (hydrophilic) is localized in the PLGA core. From the graph, it can be seen that the drug release profile of the microparticles can be controlled through changing the polymer mass ratio.

Figure 16 is a graph showing drug release profiles of a ternary phase dual-walled PLLA/PLGA/PCL microparticle. Metoclopramide HCl and Ibuprofen localized in the PLGA core and PLLA/PCL shell, respectively. From the graph, it can be seen that the release of Ibuprofen was achieved in the first 10 days, followed by Metoclopramide HCl, providing multiple drug release in a sequential manner from a single parlicle.

Figure 17 is a graph showing drug release profiles of (A) double-layered PLLA (shell)/EVA (core) microparticles, (B) double-layered PLGA (shell)/EVA (core) microparticles, (C) single-layered EVA microparticles, and (D) triple-layered PLGA(outer shell) /PLLA (middle layer) /EVA (core) microparticles. From the graph, it can be seen that the triple-layered microparticles achieved a more consistent (close to zero-order) release rate.

Figure 18 is a graph showing drug release profiles of triple layered PLGA/PLLA/EVA microparticles having mass ratio of (A) 10:2:1, and (B) 10:5:1. Ibuprofen (15 wt%) is localized in the EVA core. The graph shows that drug release profiles can be controlled through changing the polymer mass ratio (i.e. layer thickness).

Figure 19 is a graph showing drug release profiles of triple-layered of microparticles (A) PLGA (MCA in shell)/PLLA/EVA(core) and (B) PLLA (shell)/PLGA(MCA in mid-layer)/EVA(core). The graph shows that drug release profiles can be controlled through layer inversion.

## Claims

1. A method of manufacturing multi-phase microparticles having at least three polymer components, wherein the method comprises:
dissolving at least three different polymers in a volatile organic solvent to obtain a first solution, wherein the first solution comprises at least two cloud points, wherein the second cloud point is higher than the first cloud point, wherein viscosity of the first solution and first cloud point and second cloud point are selected such that the at least three different polymers become immiscible with each other upon evaporation of the volatile organic solvent;
dispersing the first solution into an aqueous continuous phase to obtain a emulsion, wherein the aqueous continuous phase comprises a surfactant; and
evaporating the volatile organic solvent from the emulsion;
wherein the total concentration of the at least three different polymers together in the emulsion before evaporation is below the first cloud point, or is above the first cloud point and below the second cloud point or is above the second cloud point, and wherein
(1) the polymers are poly(L-lactic acid) (PLLA), poly(DL-lactic-co-glycolic acid) (PLGA) and poly(caprolactone) (PCL); or
(2) wherein number of different polymers in the first solution is four and the polymers are PLLA, PLGA, polystyrene (PS) and ethylene vinyl alcohol (EVA); or
(3) the polymers are PLLA, PLGA and EVA.

2. The method of claim 1, further comprising addition of a target substance to be encapsulated to the first solution.

3. The method of any one of the preceding claims, wherein the number of different polymers in the first solution is three and the multi-phase microparticle is a ternary-phase microparticle.

4. The method of claim 3, wherein
(1) the total concentration of the three different polymers in the first solution is less than the cloud point of the polymer that has the lowest cloud point or between the cloud point of the polymer that has the lowest cloud point and the cloud point of the polymer that has the second lowest cloud point, preferably wherein the microparticle formed has a double layer structure; and
(2) the total concentration of the three different polymers in the first solution is between 2% w/v to 10% w/v ; and/or
(3) the ratio of the volatile organic solvent to aqueous continuous phase is between 0.1 to 0.005 volume of the volatile organic solvent/volume of water in v/v; or
(4) the polymer mass ratio is between 1 - 3:2 - 3:1 - 2.

5. The method of claim 3, wherein the total concentration of the three different polymers in the first solution is greater than the second cloud point, preferably wherein the microparticle formed has a triple layer structure.

6. The method of claim 1 or claim 2, wherein number of different polymers in the first solution is four and wherein the first solution thus comprises a third cloud point, wherein the total concentration of the four different polymers in the first solution is preferably greater than the third cloud point, wherein the microparticle formed has preferably a four layer structure.

7. The method of any one of the preceding claims, wherein dispersing the first solution into the aqueous continuous phase is carried out under continuous stirring, wherein the size of the microparticles formed is preferably decreased by increasing stirring speed or wherein the stirring speed is preferably between 150 to 2000 rpm.

8. The method of claim 5 or 6, wherein the ratio of the volatile organic phase to aqueous continuous phase is chosen such that the volatile organic solvent remains soluble in the aqueous continuous phase.

9. The method of any one of the preceding claims, wherein
(1) the molecular weight of each of the at least three polymers is different to each other; and/or
(2) the surfactant is an ionic surfactant or a nonionic surfactant; and/or
(3) the ionic surfactant is selected from the group consisting of sodium dodecyl sulfate (SDS), sodium pentane sulfonate, dehydrocholic acid, glycolithocholic acid ethyl ester, ammonium lauryl sulfate and other alkyl sulfate salts, sodium laureth sulfate, alkyl benzene sulfonate, soaps, fatty acid salts, cetyl trimethylammonium bromide (CTAB), dodecylethyldimethylammonium bromide (D12EDMAB), didodecyl ammonium bromide (DMAB), cetylpyridinium chloride (CPC), polyethoxylated tallow amine (POEA), hexadecyltrimethylammonium p-toluenesulfonate, benzalkonium chloride (BAC), benzethonium chloride (BZT), and mixtures thereof; and/or
(4) the nonionic surfactant is selected from the group consisting of poly(vinyl alcohol), poloaxamers, alkyl poly(ethylene oxide), diethylene glycol monohexyl ether, copolymers of poly(ethylene oxide) and poly(propylene oxide), hexaethylene glycol monohexadecyl ether, alkyl polyglucosides, digitonin, ethylene glycol monodecyl ether, cocamide MEA, cocamide DEA, cocamide TEA, fatty alcohols and mixtures thereof; and/or
(5) the volatile organic solvent has a boiling point of less than 100°C, preferably wherein the solvent is selected from the group consisting of dimethylformamide, dichloromethane, and tetrahydrofuran.

10. The method of any one of claims 2 to 9, wherein the target substance is selected from the group consisting of a drug, a protein, such as an enzyme and an antibody; a peptide, a growth factor, an organic molecule, a nucleic acid, a pesticide, a dye, and a fertilizer.

11. The method of any one of the preceding claims, wherein the polymers are biodegradable polymers or biocompatible polymers.

12. A multi-phase microparticle obtained by a method referred to in any one of the claims 1 to 11.

13. The multi-phase microparticle of claim 12 for sustained release of a target substance encapsulated therein.

14. A pharmaceutical composition comprising a multi-phase microparticle according to claim 12.

## Patentansprüche

1. Ein Verfahren zum Herstellen von Multi-Phasen-Mikropartikeln mit mindestens drei Polymerkomponenten, wobei das Verfahren umfasst:
Lösen von mindestens drei verschiedenen Polymeren in einem leicht flüchtigen organischen Lösungsmittel um eine erste Lösung zu erhalten, wobei die erste Lösung mindestens zwei Kristallisationspunkte umfasst, wobei der zweite Kristallisationspunkt höher ist als der erste Kristallisationspunkt, wobei die Viskosität der ersten Lösung und der erste Kristallisationspunkt und der zweite Kristallisationspunkt so ausgewählt sind, dass die mindestens drei verschiedenen Polymere nach Verdampfung des leicht flüchtigen organischen Lösungsmittels miteinander unmischbar werden;
Dispergieren der ersten Lösung in eine wässrige kontinuierliche Phase um eine Emulsion zu erhalten, wobei die wässrige kontinuierliche Phase einen oberflächenaktiven Stoff umfasst; und
Verdampfen des leicht flüchtigen organischen Lösungsmittels aus der Emulsion;
wobei die Gesamtkonzentration der mindestens drei verschiedenen Polymere zusammen in der Emulsion vor der Verdampfung unter dem ersten Kristallisationspunkt liegt, oder über dem ersten Kristallisationspunkt und unter dem zweiten Kristallisationspunkt liegt oder über dem zweiten Kristallisationspunkt liegt, und wobei
(1) die Polymere Poly(L-Milchsäure) (PLLA), Poly(DL-Milchsäure-co-Glycolsäure) (PLGA) und Poly(Caprolacton) (PCL) sind; oder
(2) wobei die Anzahl der verschiedenen Polymere in der ersten Lösung vier ist und die Polymere PLLA, PLGA, Polystyrol (PS) und Ethylenvinylalkohol (EVA) sind; oder
(3) die Polymere PLLA, PLGA und EVA sind.

2. Das Verfahren nach Anspruch 1, welches ferner die Addition einer Zielsubstanz umfasst, die in die erste Lösung einzubetten ist.

3. Das Verfahren nach einem der vorhergehenden Ansprüche, wobei die Anzahl der verschiedenen Polymere in der ersten Lösung drei ist und der Multi-Phasen-Mikropartikel ein Ternär-Phasen-Mikropartikel ist.

4. Das Verfahren nach Anspruch 3, wobei
(1) die Gesamtkonzentration der drei verschiedenen Polymere in der ersten Lösung niedriger ist als der Kristallisationspunkt des Polymers, das den niedrigsten Kristallisationspunkt hat, oder zwischen dem Kristallisationspunkt des Polymers, das den niedrigsten Kristallisationspunkt hat, und dem Kristallisationspunkt des Polymers, das den zweitniedrigsten Kristallisationspunkt hat, liegt, wobei das gebildete Mikropartikel vorzugsweise eine Doppelschichtstruktur hat; und
(2) die Gesamtkonzentration der drei verschiedenen Polymere in der ersten Lösung zwischen 2% Gewicht/Volumen bis 10% Gewicht/Volumen ist; und/oder
(3) das Verhältnis des leicht flüchtigen organischen Lösungsmittels zur wässrigen kontinuierlichen Phase zwischen 0.1 bis 0.005 Volumen des leicht flüchtigen organischen Lösungsmittels / Volumen von Wasser in Volumen zu Volumen ist; oder
(4) das Massenverhältnis der Polymere zwischen 1-3:2-3:1-2 ist.

5. Das Verfahren nach Anspruch 3, wobei die Gesamtkonzentration der drei verschiedenen Polymere in der ersten Lösung grösser ist als der zweite Kristallisationspunkt, wobei das gebildete Mikropartikel vorzugsweise eine Dreifach-Schichtstruktur hat.

6. Das Verfahren nach Anspruch 1 oder Anspruch 2, wobei die Anzahl der verschiedenen Polymere in der ersten Lösung vier ist und wobei die erste Lösung daher einen dritten Kristallisationspunkt umfasst, wobei die Gesamtkonzentration der vier verschiedenen Polymere in der ersten Lösung vorzugsweise grösser als der dritte Kristallisationspunkt ist, wobei das gebildete Mikropartikel vorzugsweise eine Vierfach-Schichtstruktur hat.

7. Das Verfahren nach einem der vorhergehenden Ansprüche, wobei das Dispergieren der ersten Lösung in die wässrige kontinuierliche Phase und unter kontinuierlichem Rühren durchgeführt wird, wobei die Größe der gebildeten Mikropartikel sich durch ansteigende Rührgeschwindigkeit vorzugsweise verringert oder wobei die Rührgeschwindigkeit vorzugsweise zwischen 150 und 2000 rpm beträgt.

8. Das Verfahren nach Anspruch 5 oder 6, wobei das Verhältnis der leicht flüchtigen organischen Phase zur wässrigen kontinuierlichen Phase so ausgewählt ist dass das leicht flüchtige organische Lösungsmittel in der wässrigen kontinuierlichen Phase löslich bleibt.

9. Das Verfahren nach einem der vorhergehenden Ansprüche, wobei
(1) das Molekulargewicht jedes der mindestens drei Polymere verschieden ist voneinander; und/oder
(2) das oberflächenaktive Mittel ein ionisches oberflächenaktives Mittel oder ein nichtionisches oberflächenaktives Mittel ist; und/oder
(3) das ionische oberflächenaktive Mittel ausgewählt ist aus der Gruppe bestehend aus Natriumdodecylsulfat (SDS), Natriumpentansulfonat, Dehydrocholsäure, Glycolithocholsäureethylester, Ammoniumlaurylsulfat und anderen Alkylsulfatsalzen, Natriumlaurylethersulfat, Alkylbenzolsulfonat, Seifen, Fettsäuresalzen, Cetyltrimethylammoniumbromid (CTAB), Dodecylethyldimethylammoniumbromid (D12EDMAB), Didodecylammoniumbromid (DMAB), Cetylpyridiniumchlorid (CPC), Talgfettaminethoxylat (POEA), Hexadecyltrimethylammonium-p-Toluolsulfonat, Benzalkoniumchlorid (BAC), Benzethoniumchlorid (BZT), und Mischungen davon; und/oder
(4) das nonionische oberflächenaktive Mittel ausgewählt ist aus der Gruppe bestehend aus Poly(Vinylalkohol), Poloxameren, Alkyl-Poly(Ethylenoxid), Diethylenglycolmonohexylether, Copolymere aus Poly(Ethylenoxid) und Poly(Propylenoxid), Hexaethylenglycolmonohexadecylether, Alkyl-Polyglucosiden, Digitonin, Ethylenglycolmonodecylether, Cocamid MEA, Cocamid DEA, Cocamid TEA, Fettsäurealkoholen und Mischungen davon; und/oder
(5) das leicht flüchtige organische Lösungsmittel einen Siedepunkt von weniger als 100 °C hat, wobei das Lösungsmittel vorzugsweise ausgewählt ist aus der Gruppe bestehend aus Dimethylformamid, Dichlormethan, und Tetrahydrofuran.

10. Das Verfahren nach einem der Ansprüche 2 bis 9, wobei die Zielsubstanz ausgewählt ist aus der Gruppe bestehend aus einem Medikament, einem Protein, wie zum Beispiel einem Enzym und einem Antikörper, einem Peptid, einem Wachstumsfaktor, einem organischen Molekül, einer Nukleinsäure, einem Pestizid, einem Farbstoff, und einem Düngemittel.

11. Das Verfahren nach einem der vorhergehenden Ansprüche, wobei die Polymere biologisch abbaubare oder biologisch kompatible Polymere sind.

12. Ein Multiphasen-Mikropartikel hergestellt durch das Verfahren bezüglich einer der Ansprüche 1 bis 11.

13. Der Multiphasen-Mikropartikel nach Anspruch 12 für anhaltende Freisetzung einer Zielsubstanz, die darin eingebettet ist.

14. Eine pharmazeutische Zusammensetzung umfassend einen Multiphasen-Mikropartikel gemäß Anspruch 12.

## Revendications

1. Procédé de fabrication de microparticules multiphasiques comportant au moins trois composants polymères, lequel procédé comprend :
la dissolution d'au moins trois différents polymères dans un solvant organique volatil pour obtenir une première solution, la première solution comprenant au moins deux points de trouble, le second point de trouble étant supérieur au premier point de trouble, la viscosité de la première solution et le premier point de trouble et le second point de trouble étant choisis de manière à ce que les au moins trois différents polymères deviennent immiscibles les uns aux autres suite à l'évaporation du solvant organique volatil ;
la dispersion de la première solution dans une phase continue aqueuse pour obtenir une émulsion, la phase continue aqueuse comprenant un tensioactif ; et
l'évaporation du solvant organique volatil de l'émulsion ;
dans lequel la concentration totale des au moins trois différents polymères ensemble dans l'émulsion avant évaporation est inférieure au premier point de trouble, ou est supérieure au premier point de trouble et inférieure au second point de trouble ou est supérieure au second point de trouble, et dans lequel
(1) les polymères sont le poly(acide L-lactique) (PLLA), le poly(acide DL-lactique-co-glycolique) (PLGA) et la poly(caprolactone) (PCL) ; ou
(2) dans lequel le nombre des différents polymères de la première solution est de quatre et les polymères sont le PLLA, le PLGA, le polystyrène (PS) et l'alcool éthylène vinylique (EVA) ; ou
(3) les polymères sont le PLLA, le PLGA et l'EVA.

2. Procédé selon la revendication 1, comprenant en outre l'addition d'une substance cible à encapsuler à la première solution.

3. Procédé selon l'une quelconque des revendications précédentes, dans lequel le nombre des différents polymères de la première solution est de trois et la microparticule multiphasique est une microparticule à trois phases.

4. Procédé selon la revendication 3, dans lequel
(1) la concentration totale des trois différents polymères de la première solution est inférieure au point de trouble du polymère qui a le point de trouble le plus bas ou entre le point de trouble du polymère qui a le point de trouble le plus bas et le point de trouble du polymère qui a le deuxième point de trouble le plus bas, la microparticule formée ayant de préférence une structure à deux couches ; et
(2) la concentration totale des trois différents polymères de la première solution est située entre 2 % en p/v et 10 % en p/v ; et/ou
(3) le rapport du solvant organique volatil à la phase continue aqueuse se situe entre 0,1 et 0,005 volume du solvant organique volatil/volume d'eau en v/v ; ou
(4) le rapport massique des polymères se situe entre 1 - 3/2 et 3/1 - 2.

5. Procédé selon la revendication 3, dans lequel la concentration totale des trois différents polymères de la première solution est supérieure au second point de trouble, de préférence dans lequel la microparticule formée a une structure à trois couches.

6. Procédé selon la revendication 1 ou la revendication 2, dans lequel le nombre des différents polymères de la première solution est de quatre et dans lequel la première solution comprend ainsi un troisième point de trouble, dans lequel la concentration totale des quatre différents polymères de la première solution est de préférence supérieure au troisième point de trouble, dans lequel la microparticule formée a de préférence une structure à quatre couches.

7. Procédé selon l'une quelconque des revendications précédentes, dans lequel la dispersion de la première solution dans la phase continue aqueuse est réalisée sous agitation continue, dans lequel la taille des microparticules formées est de préférence réduite en augmentant la vitesse d'agitation ou dans lequel la vitesse d'agitation se situe de préférence entre 150 et 2 000 tr/min.

8. Procédé selon la revendication 5 ou 6, dans lequel le rapport de la phase organique volatile à la phase continue aqueuse est choisi de manière à ce que le solvant organique volatil reste soluble dans la phase continue aqueuse.

9. Procédé selon l'une quelconque des revendications précédentes, dans lequel
(1) les poids moléculaires de chacun des au moins trois polymères sont différents les uns des autres ; et/ou
(2) le tensioactif est un tensioactif ionique ou un tensioactif non ionique ; et/ou
(3) le tensioactif ionique est choisi dans le groupe constitué du dodécylsulfate de sodium (SDS), du pentane sulfonate de sodium, de l'acide déshydrocholique, de l'ester éthylique d'acide glycolithocholique, du laurylsulfate d'ammonium et autres sels d'alkylsulfate, du laurethsulfate de sodium, du sulfonate d'alkylbenzène, des savons, des sels d'acides gras, du bromure de cétyl triméthylammonium (CTAB), du bromure de dodécyléthyldiméthylammonium (D12EDMAB), du bromure de didodécylammonium (DMAB), du chlorure de cétylpyridinium (CPC), de l'amine de suif polyéthoxylée (POEA), du p-toluènesulfonate d'hexadécyltriméthylammonium, du chlorure de benzalkonium (BAC), du chlorure de benzéthonium (BZT), et des mélanges de ces composés ; et/ou
(4) le tensioactif non ionique est choisi dans le groupe constitué des poloxamères de poly(alcool vinylique), de l'alkyl poly(oxyde d'éthylène), de l'éther monohexylique de diéthylène glycol, des copolymères de poly(oxyde d'éthylène) et de poly(oxyde de propylène), de l'éther monohexadécylique d'hexaéthylène glycol, des alkyl polyglucosides, de la digitonine, de l'éther monodécylique d'éthylène glycol, de la cocamide MEA, de la cocamide DEA, de la cocamide TEA, des alcools gras et des mélanges de ces composés ; et/ou
(5) le solvant organique volatil a un point d'ébullition inférieur à 100 °C, le solvant étant de préférence choisi dans le groupe constitué du diméthylformamide, du dichlorométhane et du tétrahydrofurane.

10. Procédé selon l'une quelconque des revendications 2 à 9, dans lequel la substance cible est choisie dans le groupe constitué d'un médicament, d'une protéine, telle qu'une enzyme et un anticorps ; d'un peptide, d'un facteur de croissance, d'une molécule organique, d'un acide nucléique, d'un pesticide, d'un colorant et d'un fertilisant.

11. Procédé selon l'une quelconque des revendications précédentes, dans lequel les polymères sont des polymères biodégradables ou des polymères biocompatibles.

12. Microparticule multiphasique obtenue par un procédé selon l'une quelconque des revendications 1 à 11.

13. Microparticule multiphasique selon la revendication 12 pour la libération soutenue d'une substance cible y étant encapsulée.

14. Composition pharmaceutique comprenant une microparticule multiphasique selon la revendication 12.
